# EUROPEAN PATENT SPECIFICATION

(11) **EP 2 082 234 B1**
(45) Date of publication and mention of the grant of the patent: **03.10.2012**
(21) Application number: 07827306.7
(22) Date of filing: 01.11.2007
(51) Int. Cl.: G01N 33/574

(54) **METHODS FOR SCREENING FOR THERAPEUTIC MOLECULES AND USE OF THE MOLECULES THEREFROM**
VERFAHREN ZUR SUCHE NACH THERAPEUTISCHEN MOLEKÜLEN UND VERWENDUNG DER DAMIT ERHALTENEN MOLEKÜLE
PROCÉDÉS POUR DÉPISTER DES MOLÉCULES THÉRAPEUTIQUES ET UTILISATION DES MOLÉCULES ISSUES DE CES PROCÉDÉS

(30) Priority: 02.11.2006 US 856008 P
(43) Date of publication of application: 29.07.2009
(73) Proprietor: Procognia (Israel) Ltd., Ashdod 77610 (IL)
(72) Inventor: Luria, Sylvie, Ness Ziona 74037 (IL)
(74) Representative: Becker Kurig Straus
(86) International application number: PCT/IL2007/001332
(87) International publication number: WO 2008/053486

(56) References cited:
- EP-A2- 0 171 243
- WO-A-03/074059
- US-A1- 5 330 897
- TSUCHIYA NAOTO ET AL: "Isolation and characterization of an N-linked oligosaccharide that is increased in glioblastoma tissue and cell lines" INTERNATIONAL JOURNAL OF ONCOLOGY, vol. 27, no. 5, November 2005 (2005-11), pages 1231-1239, XP008075773 ISSN: 1019-6439
- HIGUCHI MITSUNORI: "Identification of decay-accelerating factor (DAF) as a peanut aggutinin (PNA) lectin-binding molecule in human lung and its down-regulation in non-small cell lung cancers (NSCLC)." PROCEEDINGS OF THE AMERICAN ASSOCIATION FOR CANCER RESEARCH ANNUAL MEETING, vol. 44, July 2003 (2003-07), page 999, XP008075831 & 94TH ANNUAL MEETING OF THE AMERICAN ASSOCIATION FOR CANCER RESEARCH; WASHINGTON, DC, USA; JULY 11-14, 2003 ISSN: 0197-016X
- OKUYAMA NORIKO ET AL: "Fucosylated haptoglobin is a novel marker for pancreatic cancer: A detailed analysis of the oligosaccharide structure and a possible mechanism for fucosylation" INTERNATIONAL JOURNAL OF CANCER, vol. 118, no. 11, June 2006 (2006-06), pages 2803-2808, XP002471778 ISSN: 0020-7136
- BASU PRANAB S ET AL: "Immunodiagnosis of the primary brain tumor (glioma) by the endogenous lectin" CLINICA CHIMICA ACTA, vol. 317, no. 1-2, March 2002 (2002-03), pages 177-180, XP002471779 ISSN: 0009-8981
- OTAKE Y ET AL: "ISOLATION AND CHARACTERIZATION OF AN N-LINKED OLIGOSACCHARIDE THAT IS SIGNIFICANTLY INCREASED IN SERA FROM PATIENTS WITH NON-SMALL CELL LUNG CANCER" JOURNAL OF BIOCHEMISTRY, JAPANESE BIOCHEMICAL SOCIETY / OUP, TOKYO, JP, vol. 129, no. 4, 2001, pages 537-542, XP002944725 ISSN: 0021-924X
- BILYY R O ET AL: "Lectinocytochemical detection of apoptotic murine leukemia L1210 cells." CYTOMETRY. PART A : THE JOURNAL OF THE INTERNATIONAL SOCIETY FOR ANALYTICAL CYTOLOGY DEC 2003, vol. 56, no. 2, December 2003 (2003-12), pages 89-95, XP008075775 ISSN: 1552-4922
- SCHWARZ R E ET AL: "Wheatgerm agglutinin-mediated toxicity in pancreatic cancer cells" BRITISH JOURNAL OF CANCER, vol. 80, no. 11, August 1999 (1999-08), pages 1754-1762, XP002422607 ISSN: 0007-0920

## Description

### FIELD OF THE INVENTION

The present invention relates in general to the field of cancer diagnostics and treatment. In particular, the present disclosure provides methods for identifying diagnostic and therapeutic molecules that bind to a tumor-associated carbohydrate antigen on the surface of a cancer cell; pharmaceutical compositions comprising the molecules and uses thereof.

### BACKGROUND OF THE INVENTION

### Glycoconjugates

Carbohydrates comprise about one percent of the human body and can be found in free form, for example as glycosaminoglycans, or in covalent complexes (glyconjugates) with proteins (glycoproteins) or lipids (glycolipids). The carbohydrate domains of glycoproteins and glycolipids are synthesized by a series of hierarchically organized glycosyltransferases within the endoplasmic reticulum and Golgi apparatus. In addition, glycosidases can remove carbohydrates and thus modify the carbohydrate portion of the glycoconjugates (Opdenakker et al., 1993).

Glycosylation involves the addition of a carbohydrate moiety, such as a simple sugar or a glycan to a protein or lipid. Proteins and lipids can undergo glycosylation at different positions, for example, glycoproteins can carry either N-linked oligosaccharides at asparagine moieties, O-linked oligosaccharides at serine, threonine or tyrosine moieties or a combination ofN- and O-linked oligosaccharides; glycosphingolipids, consist of oligosaccharides glycosidically linked to ceramide; a ganglioside is a compound composed of a glycosphingolipid with one or more sialic acids linked on the sugar chain; and glycosylphosphatidylinositol (GPI)-linked proteins carrying a glycan chain linked to phosphatidylinositol.

Protein glycosylation can affect protein folding, intracellular trafficking and localization, the rate of degradation and can determine their organizational framework within the cytoplasm, on the membrane and extracellularly. Glycosylation of lipids was found to affect membrane rigidity and the function of membrane proteins such as growth factor receptors and integrins. Additionally, carbohydrate chains on proteins and lipids seems to play an important role in cell-to-cell interactions and interactions of cells with extra-cellular matrix and soluble molecules (Komfeld, 1980).

As vital constituents of all living systems, carbohydrates are involved in recognition, adherence, motility, and signaling processes. Glycan binding proteins (GBPs) play a significant role in decoding the information content of glycans by recognizing and specifically binding to glycosylated protein and lipid ligands. Recent development of reliable and efficient tools for analysis of GBP specificity were made using several approaches for construction of glycan arrays (see for example Blixt, et al. 2004).

### Cell Surface Carbohydrates

Alterations of cell surface carbohydrates are often observed as a result of malignant transformation and can be detected in the earliest stages of malignant transformation. The often-observed association between changes in tumor cell glycosylation and prognosis and survival of cancer patients suggests that alterations in tumor cell glycosylation patterns are an important part of tumor progression toward more malignant phenotype.

Tumors express embryonic or other specific carbohydrate antigens called tumor-associated carbohydrate antigens (TACA), which are found on both glycolipids and N- and O-linked glycoproteins and may function primarily as adhesion molecules. Some of these antigens are found exclusively in mucin-type glycoproteins and are known as T, sialyl T, Tn, and sialyl Tn, each defined by a specific monoclonal antibody. Their expression in certain types of cancer prompted the evaluation of their potential use as diagnostic and/or prognostic tools.

Gangliosides, molecules having both carbohydrate and ceramide moieties, have been identified as tumor markers for neuroectoderm derived human cancers and GD2 and GD3 are used as target molecules in antibody therapy for neuroblastomas and malignant melanomas, respectively (Houghton, et al., 1985; Kramer, et al., 2001). Recently, an anti-GD2 ganglioside antibody was shown to induce apoptosis in small cell lung cancer (SCLC) cells (Aixinjueluo, et al, 2005).

### Lectins

A family of glycan binding proteins is known as lectins. Lectins were originally isolated from various plants but have also been identified in microorganisms, insects, animals and humans (Sharon & Lis 2004). The ability of plant lectins to bind sugars and to induce aggregation of cells of various origins has been known for a century. Studies on mammalian lectins such as the galectin and selectin family of human lectins, reported no homology with plant lectins. The biological significance of these endogenous lectins is a subject of extensive investigations and it is believed that lectins play an important role in biological processes such as host defense immunity, inflammation, cell proliferation and cell death, trafficking normal leukocytes as well as malignant cells and their spread and metastatic growth (reviewed in Sharon & Lis, 2004).

Lectins exist in either soluble or cell-associated forms and possess carbohydrate-recognition domains with various specificities. The classification of lectins is based on their interaction with specific carbohydrate structures.

Non- mammalian lectins are immunogenic in nature and may bind to a host of tissue cells baring similar cell membrane associated glycan moieties. Together with the observation that human serum contains antibodies to various lectins; lectins are not considered as effective therapeutic compounds for the treatment of cancer in humans.

Various human and plant lectins have been reported to induce cell death or apoptosis of tumor cell lines. Kim et al (1993) and Gastman, et al., (2004) have shown that certain plant lectins, including Griffonia simplicifolia 1-B4 (GS1B4) and wheat germ agglutinin (WGA) lectins induce apoptosis of tumor cell lines. Zhao et al (2003) isolated a lectin, AAL, from the edible mushroom *Agrocybe aegerita* that was shown to induce apoptosis in certain tumor cell lines, including HeLa and mouse sarcoma cells.

International Patent Publication WO 97/31107 relates to human serum lectins, which selectively recognize and induce apoptosis of cells. The lectins are useful in inducing apoptosis of cancer cells whereas antibodies to the same lectins have utility in reducing xenograft rejection. That application further describes the use of anti-idiotypic antibodies directed against antibodies that antagonize the activity of the lectins. There is no teaching of apoptosis-inducing molecules that mimic the activity of plant lectins in said application.

US Patent Application Publication 20030017497 relates to methods of preparing peptide mimetopes of antigenic carbohydrate, methods of preparing recombinant antibodies and methods of use thereof for the treatment of viral, bacterial and malignant diseases by inducing an immune response to the peptide.

International Patent Publication WO 98/98535 relates to methods of treating a neoplastic disease characterized by proliferation of lymphocytes by administering pharmaceutical compositions comprising a soluble lectin isolated from the human promyelocytic leukemia cell line HL-60 or human placenta tissue. That application provides methods of treating a lectin-producing tumor by administering an effective amount of an antibody that specifically binds the lectin, lectin antagonists, inhibitory saccharides, or glycosylation inhibitors in order to prevent lectin-induced apoptosis of tumor-infiltrating T cells.

None of the above references teaches anti-cancer antibodies or other therapeutic compounds, which are characterized by their ability to displace a lectin bound to a tumor associated carbohydrate antigen or methods of identifying and using such compounds.

There remains an unmet need for compounds and compositions which selectively induce death of cancer cells.

### SUMMARY OF THE INVENTION

The present disclosure methods for identifying molecules useful in the diagnosis and treatment of diseases including cancer wherein the method is based on the displacement of exogenous lectins from cell membrane associated glycans found on cancer cells.

The present invention is based in part on the discovery that certain non-mammalian lectins selectively identify and induce death of malignant cells while leaving normal tissue unaffected. The specific binding of a lectin to a malignant cell can be disrupted by molecules having the same specificity toward the cell membrane associated glycans. Therefore, screening for molecular displacement of a specific lectin from a glycoprotein or glycolipid associated with the cell membrane of a tumor cell, provides a novel approach to identify molecules having utility in diagnosis, imaging and therapy of malignancies.

In one aspect the present disclosure provides a method for identifying a therapeutic molecule for the diagnosis or treatment of cancer, the method comprising the steps of:
a. providing a lectin which selectively binds to a tumor-associated carbohydrate antigen on a tumor cell and induces death of the tumor cell;
b. providing a library of candidate molecules;
c. contacting the tumor cell with the lectin under conditions that allow binding between the lectin and said tumor-associated,carbohydrate antigen;
d. contacting the tumor cell of (c) with the library of candidate molecules under conditions suitable to displace the lectin;
e. identifying at least one candidate molecule, which is capable of displacing the lectin.

In another aspect the lectin can be used to displace a candidate molecule. Accordingly, in another aspect the present invention provides a method for identifying a therapeutic molecule for the diagnosis or treatment of cancer, the method comprising the steps of:
a. providing a lectin which selectively binds to a tumor-associated carbohydrate antigen on a tumor cell and induces death of the tumor cell;
b. providing a library of candidate molecules;
c. contacting the tumor cell with the library of candidate molecules under conditions that allow binding between a candidate molecule and said tumor-associated carbohydrate antigen;
d. contacting the tumor cell of (c) with a lectin under suitable conditions to displace the candidate molecule;
e. identifying the candidate molecule in the displaced fraction of (d); and optionally
f. isolating the candidate molecule.

In some embodiments the library of candidate molecules is selected from the group consisting of a phage display library, a peptide library and a small compound library.

A suitable phage display library is selected from the group consisting of an antibody phage display library and a peptide phage display library.

In various embodiments the antibody phage display library displays a molecule selected from an intact antibody and an antibody fragment. An antibody fragment includes at least the antigen binding portion of an antibody and may be for example an Fab fragment or a scFv molecule.

In other embodiments the phage display library displays peptides.

In various embodiments the library of candidate molecules is a peptide library. The peptide library can comprise peptide analogs including peptidomimetics.

In some embodiments the tumor-associated carbohydrate antigen is selected from the group consisting of a glycosaminoglycan, a glycoprotein and a glycolipid. In various embodiments the glycan moiety is selected from the group consisting of sialic acid, an N-linked carbohydrate, an O-linked carbohydrate and combinations thereof.

The lectin used in methods of the invention is preferably a non-mammalian lectin. Suitable lectins include plant lectins such as Peanut Agglutinin (PNA), Ulex europaeus agglutinin (UEA), Soybean agglutinin (SBA), Dolichos biflorus (DBA), lotus tetragonolobus (LTL), Vicia villosa lectin (VVA), Maackia amurensis lectin (MAA) and Phaseolus vulgaris agglutinin (PHA); and animal lectins including Helix aspersa agglutinin (HAA) and Helix pomatia agglutinin (HPA).

In another aspect the present disclosure relates to an isolated carbohydrate binding molecule selected from the group consisting of an antibody, a peptide and a small organic compound, wherein binding of the molecule to a tumor-associated carbohydrate antigen on a cancer cell induces death of the cancer cell, and wherein said molecule is able to displace a lectin, wherein binding of the lectin to the tumor-associated carbohydrate antigen on the cancer cell induces death of said cancer cell.

In some embodiments the carbohydrate binding molecule is an antibody. According to various specific embodiments, the antibody is selected from the group consisting of: full length monoclonal antibody, chimeric antibody, humanized antibody, IgG, IgM, IgD, IgA, IgE, diabody; linear antibody and fragments thereof. According to specific embodiments, the antibody fragment is selected from the group consisting of: Fab, Fab', F(ab')₂, Fv; single-chain antibody molecules and multi-specific antibodies formed from antibody fragments.

In other embodiments the carbohydrate binding molecule is a peptide selected from the group consisting of a peptide and a peptide analog. In another embodiment the carbohydrate binding molecule is a small organic compound.

In another aspect the present disclosure provides a pharmaceutical composition comprising a molecule identified by the methods disclosed in the present invention; and a pharmaceutically acceptable excipient or carrier.

In one embodiment of the present disclosure cancer relates to the abnormal proliferative growth in of any one or more of the following organs and tissues: lung, bone, pancreatic, skin, head or neck, eye, uterus, ovary, rectum, anal region, stomach, colon, breast, fallopian tubes, endometrium, cervix, vagina, vulva, lymph including Hodgkin's and non-Hodgkin's and lymphocytic lymphomas, esophagus, small intestine, endocrine system, thyroid gland, parathyroid gland, adrenal gland, soft tissue, urethra, penis, prostate, blood including chronic or acute leukemia, bladder, kidney, the central nervous system (CNS) including spinal axis tumors, brain stem glioma; and pituitary.

In yet another aspect the present disclosure relates to a method for the treatment of cancer in a subject in need thereof the method comprising the step of:
administering to the subject a pharmaceutical composition comprising a molecule identified by the methods of the present invention.

In another aspect the present disclosure provides a method for the diagnosis of cancer in a subject in need thereof the method comprising the steps of:
a. contacting a specimen comprising suspected cancer cells isolated from the subject with a molecule identified according to the methods of the present invention; and
b. determining whether the molecule binds to the cells;
wherein detection of binding between said specimen and said molecule indicates a positive diagnosis of cancer.

In some embodiments of the disclosure provides a method for the diagnosis of non-small cell lung carcinoma (NSCLC) in a subject in need thereof the method comprising the steps of:
a. contacting a specimen comprising suspected NSCLC cells from the subject with a non-human lectin selected from the group consisting of PNA, DBA, UEA, SBA, LTL, VVA, MAA, and PHA; and
b. determining whether the lectin binds to the cells;
wherein detection of binding between said cells and said molecule indicates a positive diagnosis of cancer.

A suitable specimen is selected from the group consisting of tissue biopsies, e.g. breast tissue, colorectal tissue, pleural tissue, pancreatic tissue, brain, hepatic tissue, gastrointestinal tissue, bladder tissue, dermal tissue and ovarian tissue.

In some embodiments the specimen is a body fluid selected from urine, blood, cerebrospinal fluid and saliva. In various embodiments the fluid is serum.

Further embodiments and the full scope of applicability of the present invention will become apparent from the detailed description given hereinafter.

It is to be explicitly understood that known carbohydrate-binding molecules are excluded from the present invention, however, novel uses of known molecules in methods of diagnosing and killing cancer cells are within the scope of the present invention.

### BRIEF DESCRIPTION OF THE FIGURES

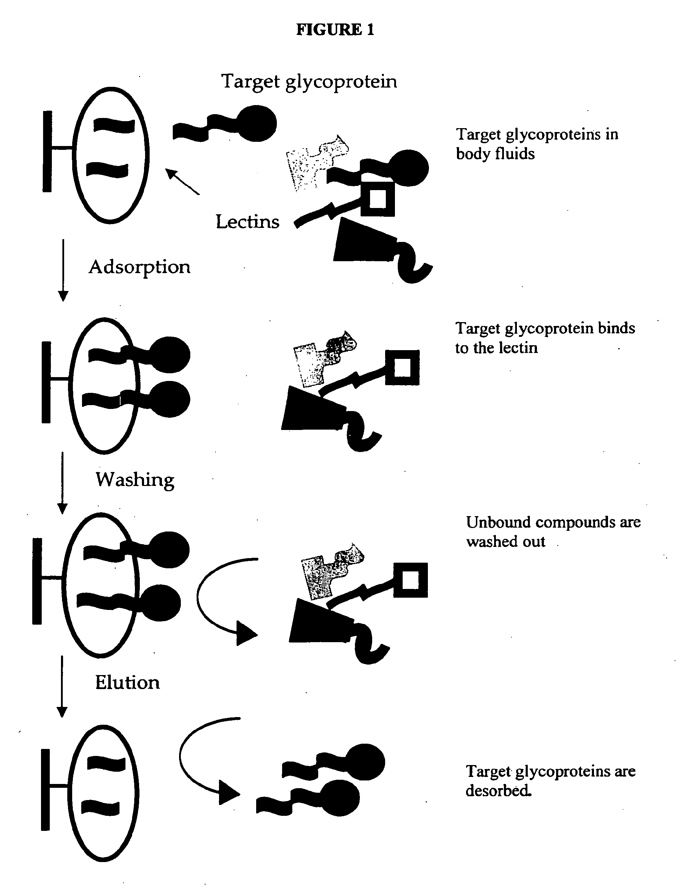

Figure 1 outlines the process of lectin affinity separation.

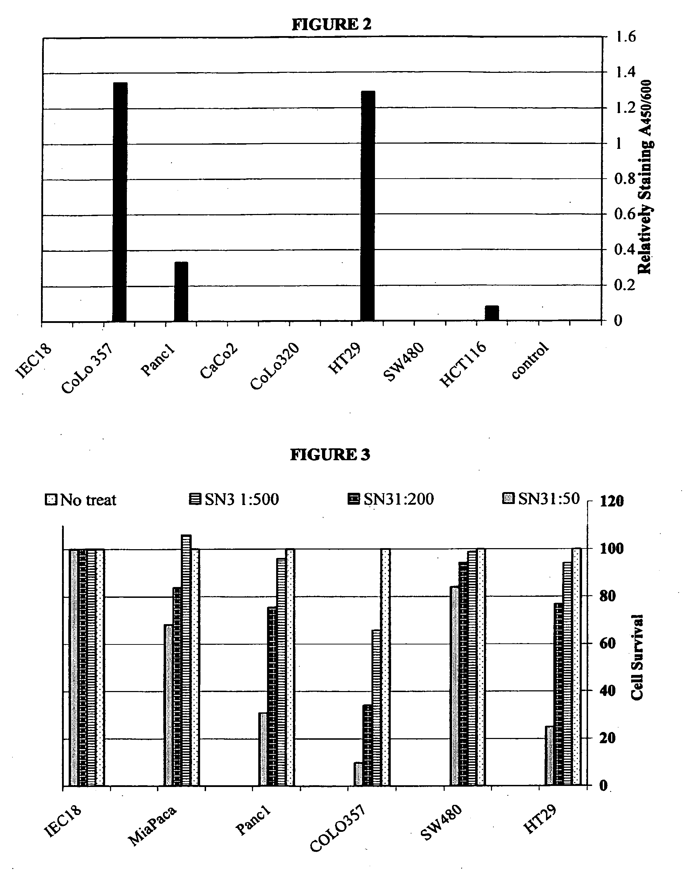

Figure 2 shows a graph of the relative staining of different cancer cell lines with the SN3 antibody. Colo357 and Panc1 are pancreatic cancer lines; CaCo2, CoLo320 HT29, SW-480, HCT116 are colon cancer cell lines; Rat-EC18 is a normal rat colon cell line.
Figure 3 shows the survival of cancer cell lines following treatment with SN3 antibodies. The cell lines are the same as in figure 2.

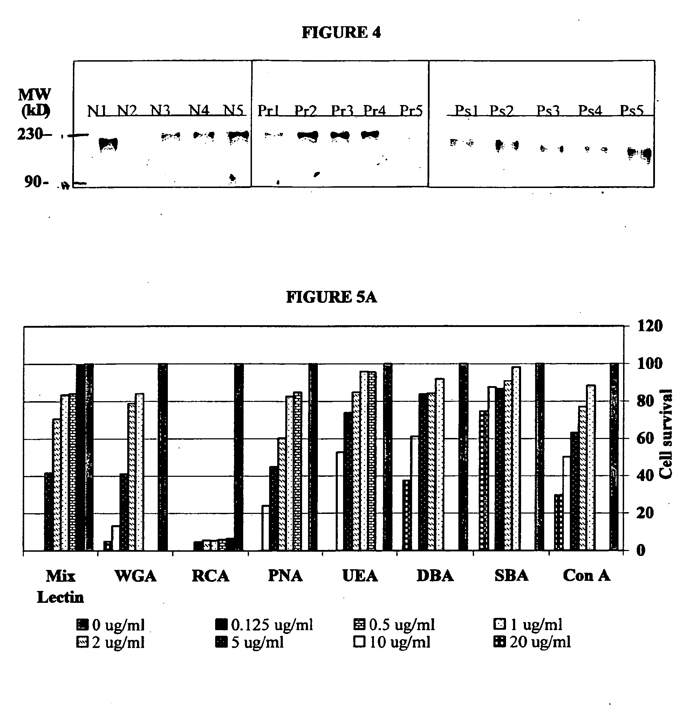

Figure 4 is a figure of a Western blot labeled with anti-Muc I antibodies. Muc-I was isolated from human sera by a mixture of lectins and identified using an anti-Muc 1 antibody. The lanes are normal serum (age >50 years) (N), breast cancer sera pre-operation (Pr), sera from reoccurrence breast cancer patients (Ps).
Figures 5A-5C show cell survival of normal skin fibroblasts (5A), NSCLC H1299 cells (5B) and NSCLC A549 cells (5C) upon treatment with varying doses of the lectins WGA,RCA, PNA, UEA, DBA, SBA, Con A and mixture thereof.

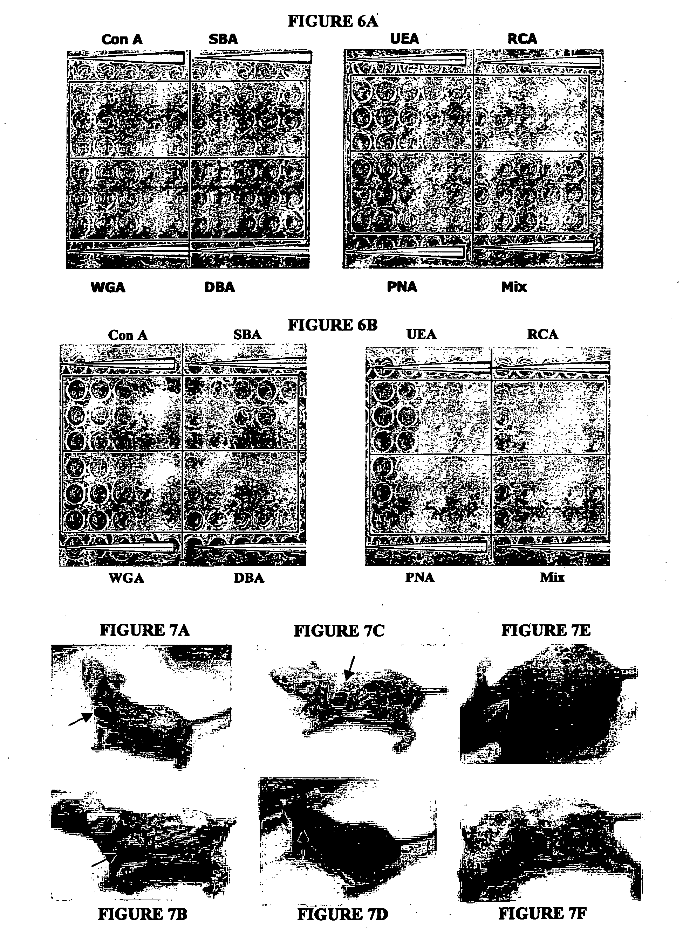

Figure 6: Methylene blue assay. The wide side of the arrow represents an increasing concentration of lectin. The methylene blue assay correlates with cell survival, since it binds DNA in basic condition and is eluted in acid condition.
Figures 7A-7F show the effect of lectins on tumors induced in mice. Figures 7A and 7B show tumors in mice following injection with human H1299 non small cell lung cancer cells, figures 7C and 7D show the tumors in mice injected with human H1299 non small cell lung cancer cells and treated five days later with a mixture of lectins (PNA, UEA & SBA) injected locally into the site of tumor cell injection, figures 7E and 7F show control mice injected with the lectin mixture only.

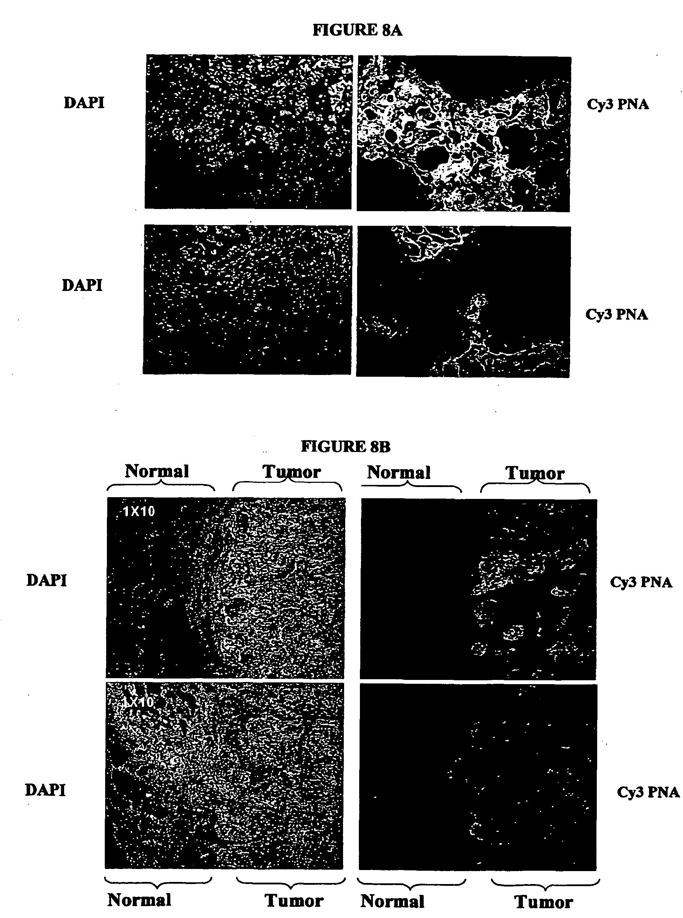

Figures 8A and 8B are micrographs immunohistochemically stained human lung tissue sections. Figure 8A shows DAPI (left panels) and Cy3 labeled PNA (biotinylated PNA and streptavidin Cy3; right panels) staining. Figure 8B shows tissue sections, which include both normal and tumor (NSCLC) tissue. The tumor tissue is specifically stained.

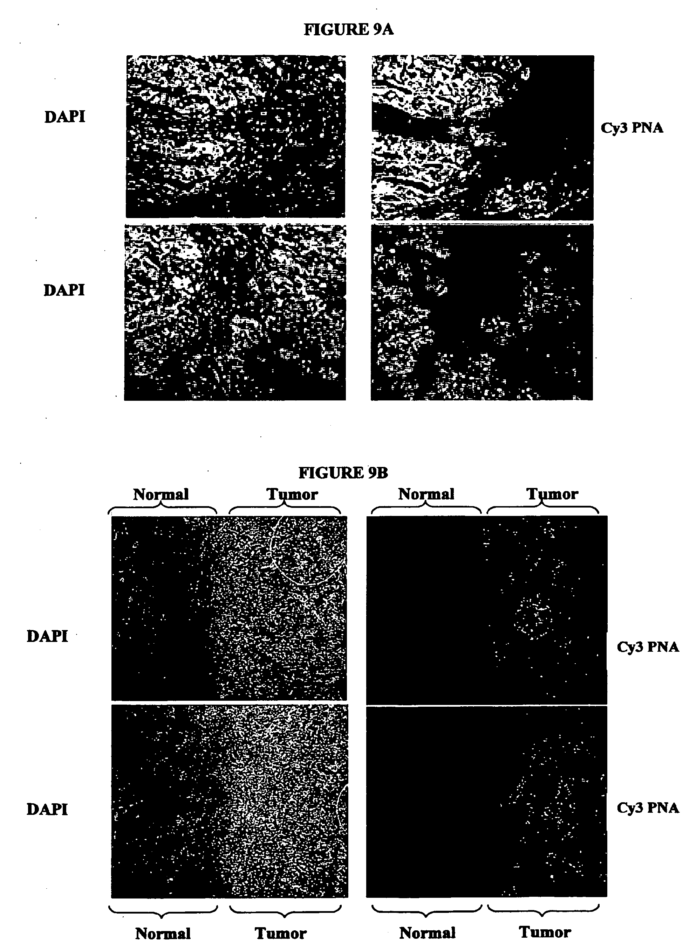

Figures 9A and 9B are micrographs of immunohistochemically stained human lung tissue sections from a patient different then those of figure 8. Figure 9A shows DAPI (left panels) and Cy3 labeled PNA (biotinylated PNA and streptavidin Cy3; right panels) staining. Figure 9B shows sections, which include both normal and tumor (NSCLC) tissue. The tumor tissue is specifically stained.

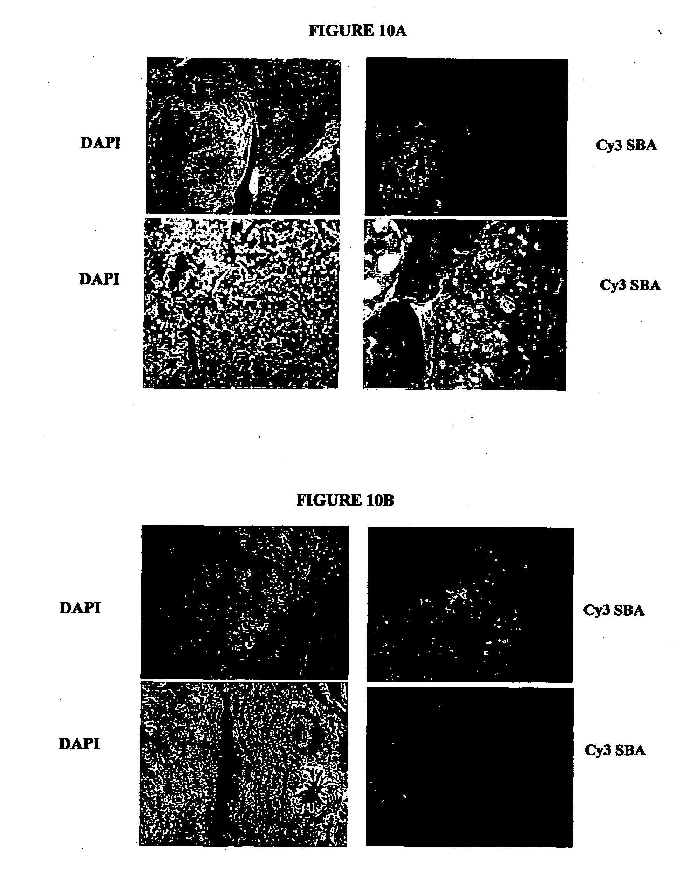

Figures 10A and 10B show DAPI (left panels) and Cy3 labeled SBA (biotinylated SBA and streptavidin Cy3; right panels) staining. Figure 10A shows the staining of a lung section from a patient with NSCLC. Figure 10B shows absence of SBA staining in normal lung tissue (DAPI: left panels, SBA-Cy3: right panels).

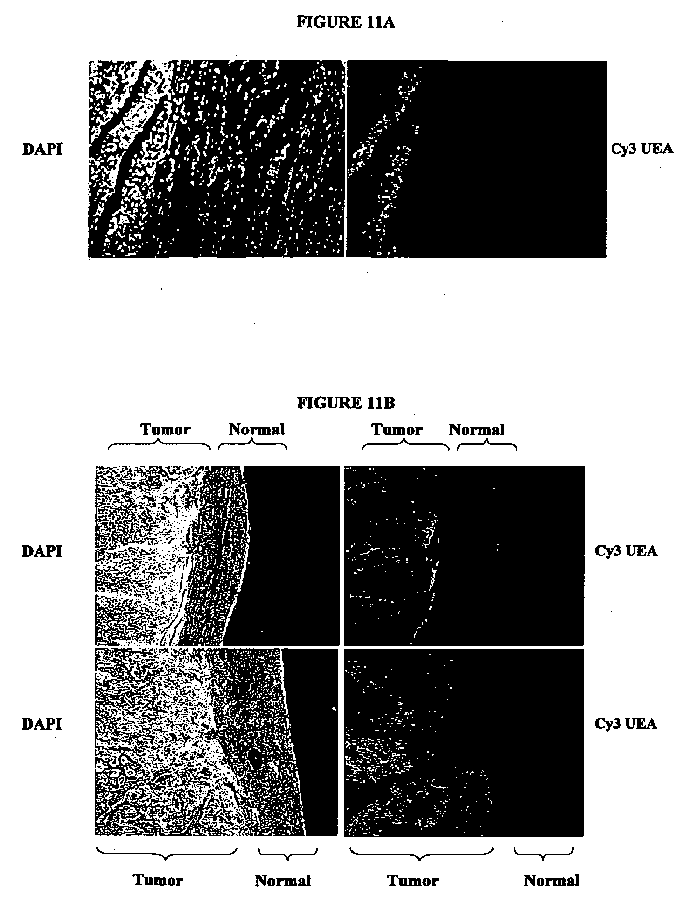

Figures 11A and 11B show DAPI (left panels) and Cy3 labeled UEA (biotinylated UEA and streptavidin Cy3; right panels) staining. Figure 11A shows the staining of a lung section from a patient with NSCLC adenocarcinoma. Figure 11B shows tissue sections which include both normal and tumor (NSCLC) tissue. The tumor tissue is specifically stained.

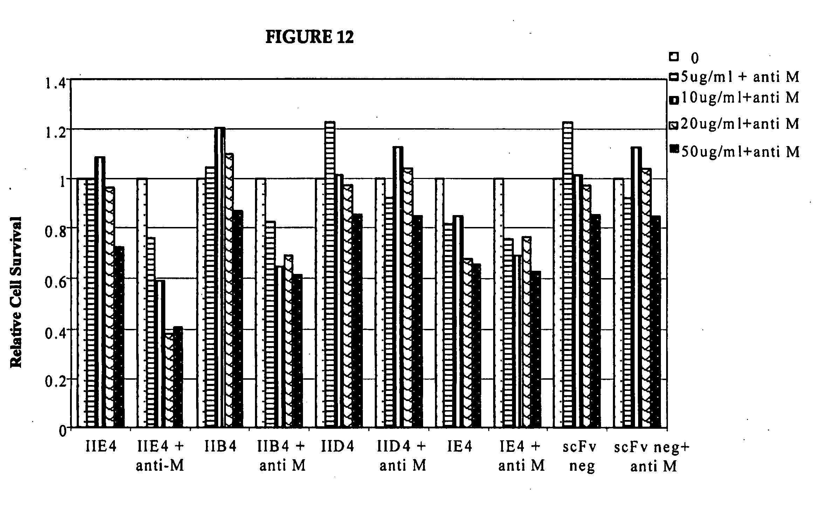

Figure 12 shows a graph of A549 NSCLC adenocarcinoma cell growth inhibition by the isolated scFv fragments, either as monovalent fragments or bivalent fragments cross-linked with the secondary antibody, using methylene blue assay.

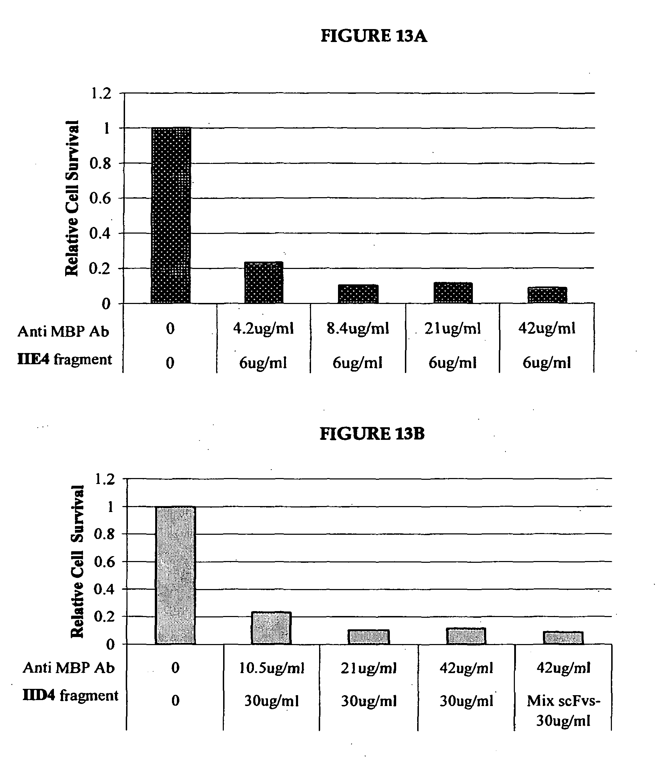

Figures 13A and 13B show graphs of A549 NSCLC adenocarcinoma cell growth inhibition by the scFv IIE4 and scFv IID4 fragments by titrating the secondary antibody. Figure 13A shows A549 NSCLC adenocarcinoma cell growth inhibition by the scFv IIE4 fragment. Figure 13B A549 NSCLC adenocarcinoma cell growth inhibition by the scFvIID4 fragment. A 1:1 ratio of the fragments with the secondary antibody generate a bivalent binder that induces cell apoptosis.

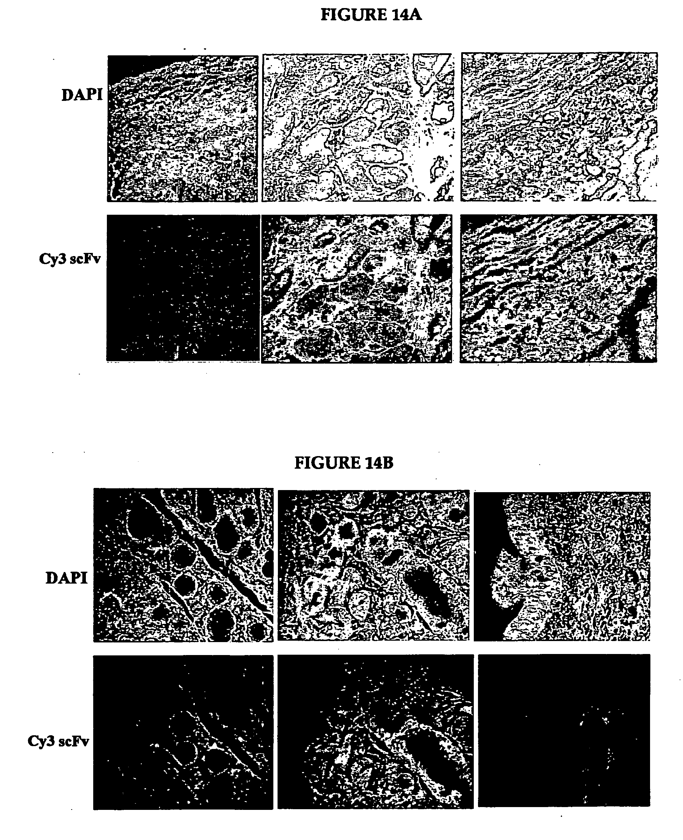

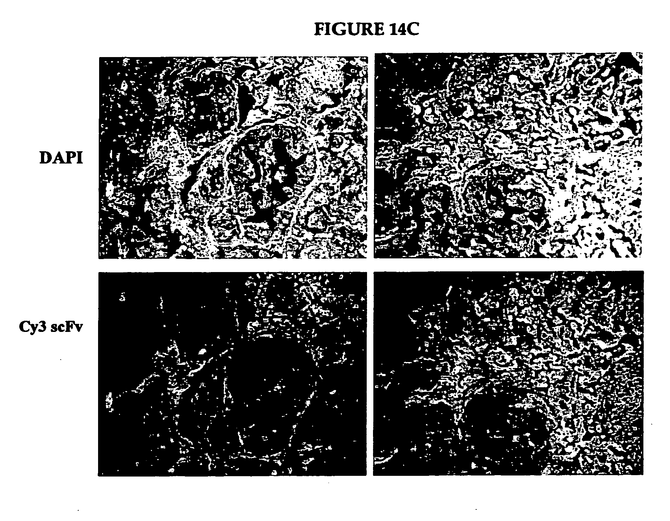

Figures 14A, 14B and 14C show imunohistochemical staining of human NSCLC specimens with scFv IIE4 fragment using secondary anti maltose binding protein conjugated to Cy3. DAPI staining (upper panels) was used to identify the tissue. The tissue sections include both normal and tumor (NSCLC) tissue. The tumor tissue is specifically stained. Figure 14A shows staining of human NSCLC specimen of case number 18828.00, control staining with the secondary Cy3 alone is shown in the left panel; figure 14B shows staining of human NSCLC specimen of case number 3320.02; figure 14C shows staining of human NSCLC specimen of case number 16317.02.

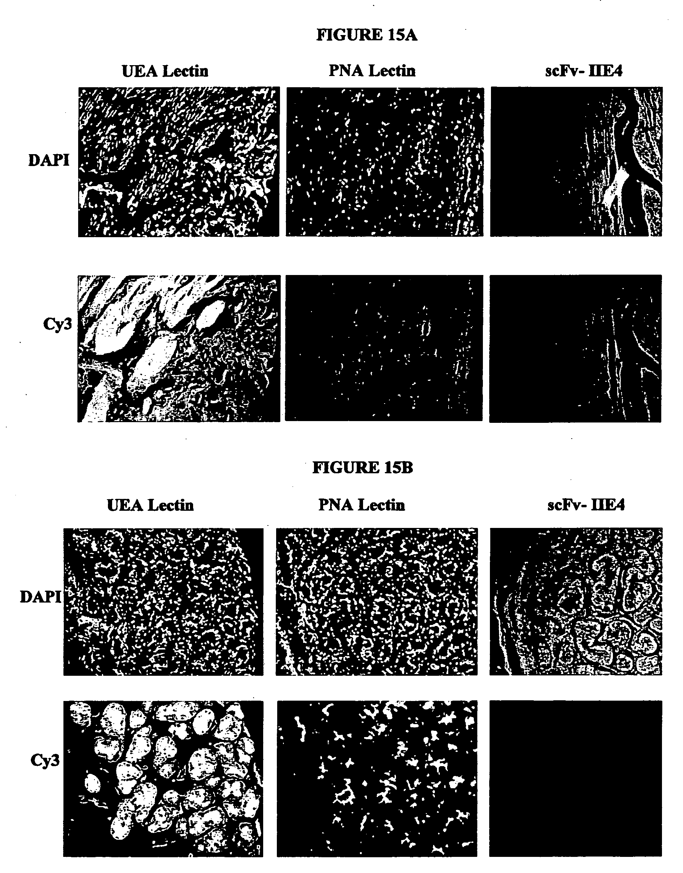

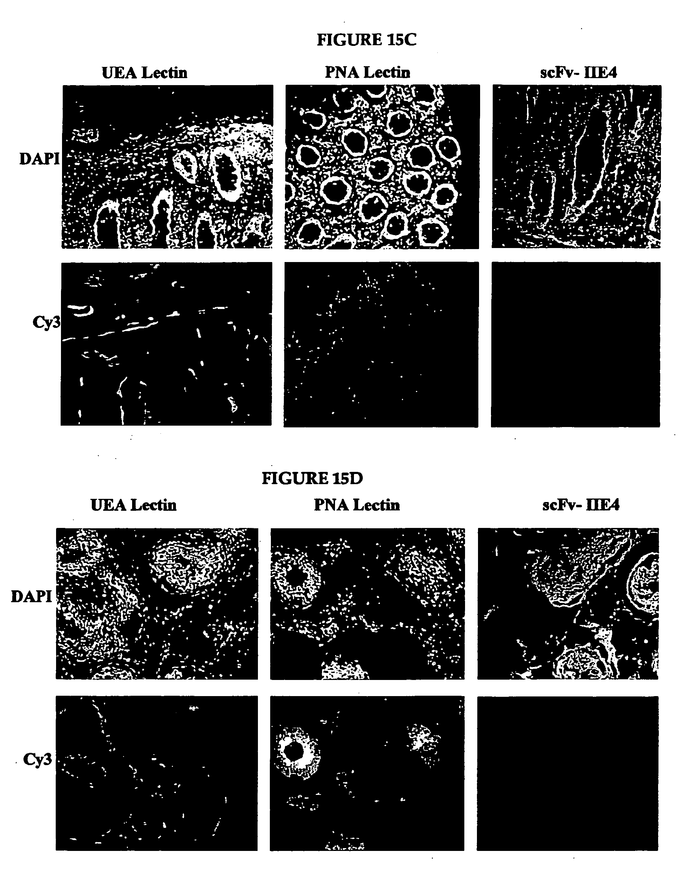

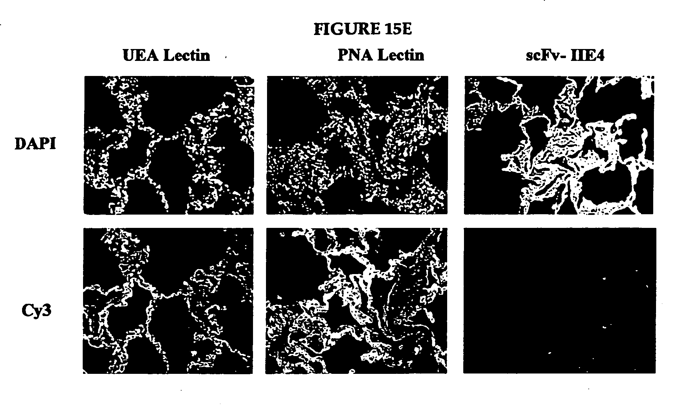

Figures 15A-15E show normal human tissues array paraffin embedded slides that were stained with scFv IIE4 fragment using secondary anti maltose binding protein conjugated to Cy3, with PNA and EUA lectins conjugated to Cy3. DAPI staining was used to identify the tissue. The results demonstrated that PNA and EUA lectins stain normal tissue in different pattern, however, staining of the normal tissue array with scFv IIE4 fragment demonstrated negative staining. Figure 15A shows staining of normal human gallbladder tissue, figure 15B shows staining of normal human stomach tissue, figure 15C shows staining of normal colon tissue, figure 15D shows staining of normal human testis tissue, figure 15E shows staining of normal lung tissue.

### DESCRIPTION OF THE INVENTION

The present disclosure relates to methods and tools for diagnosing cancers by identifying molecules that are able to displace lectins, which bind specifically to cancer cells and induce their death. The present invention is based in part on the discovery that certain non-human lectins are able to selectively bind to tumor cells and induce their death while having no effect on the neighboring normal cells. This discovery has enabled the development of a screening method for the identification of molecules useful in diagnosis, imaging and therapy of certain malignancies. The identified molecules act as carbohydrate binding molecules and inducers of cell death, including apoptosis, and have been designated herein as "LICAs", an acronym for "Ligand Induced Cell Apoptosis" agent.

Additionally, the present disclosure also relates to diagnostic and therapeutic methods, the methods and identified molecules will help fill the following diagnostic and therapeutic gaps:
early diagnosis of slow-growing difficult-to-detect tumors characterized by fewer early symptoms such as adenocarcinoma, by blood serum testing, tissue staining and in-vivo imaging.
specific targeting of such tumors by therapeutic compositions to trigger tumor-selective cell death
classification of tumor to tumor classes and response to therapy, and
monitoring of therapy.

The present invention is based in part on the discovery that carbohydrate moieties (tumor-associated carbohydrate antigens; TACA) are found on the surface of cancer cells are sensed by antibodies and by specific non-human lectins and induce cell death. Without wishing to be bound to theory, cancer cells are tagged for elimination by a unique and general mechanism that senses abnormal cells for destruction. Cancer develops over time upon an imbalance caused by a variety of molecular and genetic abnormalities, leading to the accumulation of cancer cells and the appearance of the disease.

A group of non-human lectins that selectively bind to cancer cells and deliver a selective death signal to the cancer cells but not to normal cells has been identified. The lectins having selectivity in binding and killing cancer cells include: PNA, DBA, UEA, SBA, LTL, VVA, MAA, and PHA.

The above lectins were analyzed and found to have the following activities:
1. The lectins selectively bind to cancer cells in-vitro;
2. The lectins induce selective death induced signal in cancer cells;
3. The lectins reduce tumor progression in tumor bearing animal model;
4. The lectins selectively stain tissue of affected individuals diagnosed with adenocarcinoma of non-small cell lung carcinoma.

### Definitions

For convenience certain terms employed in the specification, examples and claims are described herein.

The term "tumor-associated carbohydrate antigen" refers to a carbohydrate moiety that is linked to the cell membrane of a tumor cell either directly or via another molecule such as a protein or lipid. Thus, the carbohydrate can be a polysaccharide as well as a molecule to which a polysaccharide is linked (e.g., by a covalent bond) to a second molecule. The tumor-associated carbohydrate antigen may be a lectin, a glycosaminoglycan, a glycoprotein or a glycolipid. Carbohydrate antigens are conjugated to lipids and proteins via a process known as glycosylation.

The term "selectively" and "specificity" refers to the ability to have an effect on a one type of cell without manifesting an effect on a second type of cell. In particular, it refers to the ability to affect cancer cells while having no effect on normal cells and tissues.

The term "displaces" refers to the partial or complete removal of a molecule from its bound position. As used herein, the term "bind" or "binding" refers to the association of a molecule with a carbohydrate associated with the cell membrane to form a bound complex. In particular, the binding of a lectin, antibody or fragment thereof, peptide or peptide analog or small organic compound to the carbohydrate domain of a glycoprotein, glycolipid or glycosaminoglycan is sufficiently specific such that the bound complex can form *in vivo* in a cell or *in vitro* under suitable conditions.

As used herein, the term "carbohydrate binding molecule" refers to a molecule including a lectin, antibody or a fragment thereof, a peptide, a peptide analog and a small organic and inorganic molecule to bind to a carbohydrate moiety associated with the cell membrane wherein that binding depends on the specific structure and properties of the molecule.

As used herein "cancer" refers to tumor formation, primary tumors, tumor progression and tumor metastasis. Cancer includes for example breast cancer, lung cancer, prostate cancer, colorectal cancer, brain cancer, esophageal cancer, stomach cancer, bladder cancer, pancreatic cancer, cervical cancer, head and neck cancer, ovarian cancer, melanoma, lymphoma, glioma, or multi-drug resistant cancer.

The term "metastatic disease" includes a tumor having metastatic potential as well as tumor metastases, *per se.* Specifically metastatic disease refers to cancers having a metastatic potential and to metastases that have spread to regional lymph nodes or to distant sites. In preferred embodiments of the present disclosure metastatic disease refers to colorectal cancer, prostate cancer, ovarian cancer, non-small cell lung cancer and hepatocellular cancer and the metastases derived therefrom. The most common sites for colon cancer metastasis, for example, are lymph node, lung, bone and liver.

### Inducting cell death for cancer therapy

The present disclosure provides a carbohydrate binding molecule which is able to induce death of a cancer cell comprising on its surface a tumor-associated carbohydrate antigen. Cell death as used herein refers to apoptosis or necrosis. Anoikis is apoptosis induced by loss of cell anchorage.

Apoptosis represents a universal and exquisitely efficient cellular suicide pathway. Apoptosis has vital role in normal development, and numerous genes have been identified that encode apoptotic regulators, some of which represent familiar oncogenes or tumor-suppressor genes. Because of the convergence between cancer biology and cell death regulation in development, there is a great focus on molecular pathways whose endpoint, death, coincides with the goal of successful treatment. Apoptotic cell death is triggered by intracellular cues such as DNA damage and osmotic stress, and extracellular cues including growth factor withdrawal, matrix detachment, and direct cytokine-mediated killing. Two central pathways are involved in the process of apoptotic cell death, one involving the activation of the caspase proteases and a second, mitochondrial, pathway. A common feature of this machinery and of the signaling pathways that impinge on this machinery is that at nearly every level, the action of proapoptotic molecules is opposed by sets of inhibitors.

A number of death pathways converge on the caspase cascade. These pathways begin when a death ligand such as TNF or FasL interacts with its cognate receptor, TNF-R or Fas (CD95), inducing the trimerization of the receptors. Receptors in this family (TNF-R1, Fas, DR3, DR4, DR5, and DR6) all contain an intracellular "death domain," and receptor trimerization recruits adaptor proteins such as FADD to this domain. These adaptor molecules then recruit and activate caspase 8, although in certain instances caspase 8 may be recruited directly to the receptor. The death receptors are countered by a number of decoy receptors, which lack the death domain and may antagonize death receptor activation. A second, mitochondrial pathway to apoptosis is the Bcl-2 family of proteins. The antiapoptotic protein Bcl-2, is the founding member of a large family that consists of proapoptotic factors such as Bax, Bak, Bcl-XS, and Bad, and antiapoptotic factors such as Bcl- 2, Bcl-XL, and Bcl-W.

In cancer, the therapeutic goal is to trigger tumor-selective cell death. The mechanisms responsible for such death are of obvious importance in determining the efficacy of specific treatments.

Apoptosis as a therapeutic goal offers advantages over nonapoptotic death mechanisms only if the therapeutic index or the availability of compounds that induce it is greater. In drug-curable malignancies, such as common pediatric leukemias and certain solid tumors, apoptosis is a prominent (if not the exclusive) mechanism associated with the induction of tumor remission. In addition, the expression of apoptotic modulators within a tumor appears to correlate with its sensitivity to traditional cancer therapies. No strict correlation between the induction of apoptosis and a patient's long-term prognosis has emerged, perhaps in part because the ability to achieve initial remission alone does not adequately predict long-term outcome.

Alternatively, one could activate the death pathways directly, using soluble death ligands. Thus far, this approach has been problematic, because administered TNFα and FasL are each broadly cytotoxic.

### Library Screening

According to one embodiment the present disclosure provides a method of identifying therapeutic and diagnostic molecules which displace lectin binding to cancer cells, wherein the method comprises screening an antibody, peptide and or small molecule library. Libraries comprising those molecules are known to those with skill in the art.

In recent years, a large number of combinatorial chemistry techniques have been developed which permit vast libraries of diverse chemical compounds to be rapidly synthesized. For example, Chemical Diversity Labs Inc. (San Diego, CA), a supplier of chemical compounds, released a database named CombiLab Probe Libraries listing 220,674 compound structures; I.F. Lab (Kiev, Ukraine), a supplier of chemical compounds, released a database named IF LAB Libraries listing 77,098 compound structures. The ability to rapidly generate large collections of compounds using combinatorial chemistry techniques has created a need for new methods of screening compound libraries. The traditional approach of screening each compound individually in an assay to identify those compounds having the desired biological activity is no longer practical due to time and resource constraints. There remains a need for new methods which permit the rapid screening of compound libraries for compounds having an activity of interest. The compounds are also referred to as small organic molecules.

Random peptide libraries are conveniently assembled by chemical synthesis as a collection of peptide sequences in which all amino acids have been incorporated randomly into all positions of the peptide. Each peptide in the mixture will have a unique sequence and all possible sequences for a residue peptide will be represented. Such libraries have been generated and used in various ways to screen for peptide sequences which bind effectively to target molecules and to identify such sequences. A variety of methodologies can be applied to the problem of generating a diverse group of candidate compounds, based on the known principles of peptide chemistry and/or molecular biology. Such synthetic methods for the preparation of random peptide libraries are well known in the art. Related patents include US Patents 6,008,058; 6,194,544; 6,117,974; EP 06395841B1 and the like.

Random peptide libraries may also be formed by random nucleotide sequences. One method of randomizing the nucleotide sequences is the addition of equal proportions of all four nucleotides in the monomer coupling reactions. The resulting random incorporation of all nucleotides yields a population of oligonucleotides coding random amino acid sequences. However, this approach has a built-in bias due to the redundancy of the genetic code. The amino acid bias can be alleviated by synthesizing the DNA from nucleotide triplets. Here, a triplet coding for each of the twenty amino acids is synthesized from individual monomers. Once synthesized, the triplets are used in the coupling reactions instead of individual monomers. By mixing equal proportions of the triplets, synthesis of oligonucleotides with random codons can be accomplished.

Over recent years, the use of phage-display technology to produce and screen libraries of peptides and polypeptides for binding to a selected target has expanded. Related patents include US Patents 5,702,892; 6,696,248 and 6,794,128 and the like.

A phage particle displays a polypeptide as part of a capsid enclosing the phage genome which encodes the polypeptide. This technology allows simultaneous mass screening of very large numbers of phage bearing different polypeptides. Phage displaying a polypeptide with affinity to a target binds to the target and the phages can be enriched by affinity screening to the target. The identity of peptides and polypeptides displayed from the phage can be determined from their genomes and a peptide or polypeptide identified as having a binding affinity for a desired target can then be synthesized in large scale (Benhar, 2001).

In some embodiments the preferred molecule is an antibody fragment. Phage display technology has also been used to produce and screen libraries of heterodimeric proteins, such as Fab fragments. In general a phage-Fab fragment has one antibody chain fused to a phage coat protein so that it is displayed from the phage coat and the other antibody chain is complexed with the first chain, although other techniques are possible. For example, US Patent 6,706,484, provides methods the preparation of a libraries of human antibodies and antibody fragments by the use of synthetic consensus sequences which cover the structural repertoire of antibodies encoded in the human genome. As exemplified herein below the antibody fragment may be selected by screening of a human scFv phage display library derived from the rearrangement of a V-gene repertoire of adult human donors (Azriel-Rosenfeld et al., 2004).

Additionally peptide and polypeptide libraries have been displayed from replicable genetic forms other than phage including eukaryotic viruses and bacteria. The principles and strategy are closely analogous to those employed for phage, namely, that nucleic acids encoding antibody chains or other polypeptides to be displayed are inserted into the genome of the package to create a fusion protein between the polypeptides to be screened and an endogenous protein that is exposed on the cell or viral surface. Expression of the fusion protein and transport to the cell surface result in display of polypeptides from the cell or viral surface. For example, US Patent 7,125,973, provides methods and vectors for the display of recombinant proteins on eukaryotic host cell surface by providing a fusion protein with an extracellular anchor.

### Antibodies

The term "antibodies" includes molecules having the antigen-binding portion of an antibody, intact immunoglobulin molecules of any isotype and generated by any animal, cell line or microorganism, polyclonal or monoclonal antibody, and proteolytic fragment thereof. According to various specific embodiments, the monoclonal antibody may be selected from the group consisting of: full length monoclonal antibody, chimeric antibody, humanized antibody, IgG, IgM, IgD, IgA, IgE, diabody; linear antibody and fragments thereof. According to particular embodiments, the antibody fragment is selected from the group consisting of: Fab, Fab', F(ab')₂, Fv; single-chain antibody molecules and multi-specific antibodies formed from antibody fragments.

A molecule having the antigen-binding portion of an antibody as used herein is intended to include the antigen-binding reactive fraction thereof, including, but not limited to, the Fab fragment, the Fab' fragment, the F(ab')₂ fragment, the variable portion of the heavy and/or light chains thereof, Fab miniantibodies, dimeric bispecific miniantibodies and engineered antibodies such as chimeric or single-chain antibodies incorporating such reactive fraction, as well as any other type of molecule or cell in which such antibody reactive fraction has been physically inserted, such as a chimeric T-cell receptor or a T-cell having such a receptor, or molecules developed to deliver therapeutic moieties by means of a portion of the molecule containing such a reactive fraction. Such molecules may be provided by any known technique, including, but not limited to, enzymatic cleavage, peptide synthesis or recombinant techniques.

Antibodies, or immunoglobulins, comprise two heavy chains linked together by disulfide bonds and two light chains, each light chain being linked to a respective heavy chain by disulfide bonds in a "Y" shaped configuration. Proteolytic digestion of an antibody yields Fv (fragment variable), Fab fragments and Fc (fragment crystalline) domains, depending on the proteolytic enzyme. The antigen binding domains, Fab, include regions where the polypeptide sequence varies. The term F(ab')₂ represents two Fab' arms linked together by disulfide bonds. The central axis of the antibody is termed the Fc fragment. Each heavy chain has at one end a variable domain (V_{H}) followed by a number of constant domains (C_{H}). Each light chain has a variable domain (V_{L}) at one end and a constant domain (C_{L}) at its other end, the light chain variable domain being aligned with the variable domain of the heavy chain and the light chain constant domain being aligned with the first constant domain of the heavy chain (CH1).

The variable domains of each pair of light and heavy chains form the antigen-binding site. The domains on the light and heavy chains have the same general structure and each domain comprises four framework regions, whose sequences are relatively conserved, joined by three hypervariable domains known as complementarity determining regions (CDR1-3). These domains contribute specificity and affinity of the antigen-binding site. CDR grafting may be performed to alter certain properties of the antibody molecule including affinity or specificity. A non-limiting example of CDR grafting is disclosed in US Patent 4,946,778.

The isotype of the heavy chain (gamma, alpha, delta, epsilon or mu) determines immunoglobulin class (IgG, IgA, IgD, IgE or IgM, respectively). The light chain is either of two isotypes (kappa, κ or lambda, λ) found in all antibody classes.

The term "Fc" as used herein is meant as that portion of an immunoglobulin molecule (Fragment crystallizable) that mediates phagocytosis, triggers inflammation and targets Ig to particular tissues; the Fc portion is also important in complement activation.

An "antigen" is a molecule or a portion of a molecule capable of being bound by an antibody which is additionally capable of inducing an animal to produce antibody capable of binding to an epitope of that antigen. An antigen may have one or more than one epitope. The specific reaction referred to above is meant to indicate that the antigen will react, in a highly selective manner, with its corresponding antibody and not with the multitude of other antibodies which may be evoked by other antigens. The antigen of the present disclosure includes at least one carbohydrate moiety.

A "monoclonal antibody" or "mAb" is a substantially homogeneous population of antibodies to a specific antigen. mAbs may be obtained by methods known to those skilled in the art. See, for example Kohler et al (1975); US Patent 4,376,110; Ausubel et al (1987-1999). The mAbs of the present disclosure may be of any immunoglobulin class including IgG, IgM, IgE, IgA, and any subclass thereof. A hybridoma producing a mAb may be cultivated *in vitro* or *in vivo.* High titers of mAbs can be obtained by *in vivo* production where cells from the subject hybridomas are injected intraperitoneally into pristine-primed Balb/c mice to produce ascites fluid containing high concentrations of the desired mAbs. mAbs of isotype IgM or IgG may be purified from such ascites fluids, or from culture supernatants, using column chromatography methods well known to those of skill in the art.

The use of recombinant human monoclonal antibodies is becoming increasingly popular due to their advantages over conventional monoclonal or polyclonal antibodies. For instance, as regulations for experiments animal use become more stringent, the production of recombinant antibodies requires no animals. The production or isolation of recombinant antibodies is much faster than conventional antibody production and they can be generated against an enormous number of antigens. In contrast, in the conventional method, many antigens prove to be non-immunogenic or extremely toxic, and therefore do not generate antibodies in animals. Moreover, affinity maturation (i.e. increasing the affinity and specificity) of recombinant antibodies is very simple and relatively fast. Finally, large numbers of different antibodies against a specific antigen can be generated in one selection procedure.

An antibody library can be generated using several technologies:

A synthetic antibody repertoire can be generated by cloning synthetic CDR3 regions in a pool of heavy chain germline genes and thus generating a large antibody repertoire, from which recombinant antibody fragments with various specificities can be selected (Nissim et al., 1994; Griffiths et al., 1994).

Alternatively, a human lymphocyte pool can be used as starting material for the construction of an antibody library. It is possible to construct naive repertoires of human IgM antibodies, thus creating a human library of large diversity. This method has been used successfully to select a large number of antibodies against different antigens by Marks et al. (1991).

Another possibility is to prepare so-called patient libraries. First, the sera of individuals are tested for the presence of specific antibodies directed to the antigen of interest. From the lymphocyte pool of positive individuals, IgG libraries can be generated, which will contain IgG antibodies of high specificity and with high affinity.

Finally, construction of specialized libraries will enable us to generate antibodies for studying specific biomolecules e.g. libraries from patients with certain tumors for selection of tumor markers, or libraries adapted for the generation of antibodies against specific molecules such as sugar residues.

Once libraries are generated, they can be used for selection of recombinant monoclonal antibodies against various antigens, by immobilizing the antigen of interest and applying various selection rounds with the appropriate phage display libraries. The screening protocol can combine differential and subtractive screening, by comparing different tissues, cells or organisms.

Phage display technology is much faster than conventional antibody production and antibodies can be generated against an enormous number of antigens. Moreover, affinity maturation (i.e., increasing the affinity and specificity) of recombinant antibodies is very simple and relatively fast. Finally, large numbers of different antibodies against a specific antigen can be generated in one selection procedure. To generate recombinant monoclonal antibodies one can use various methods all based on phage display libraries to generate a large pool of antibodies with different antigen recognition sites. Protocols for bacteriophage library construction and selection of recombinant antibodies are provided in the reference text Current Protocols in Immunology, Colligan et al (Eds.), John Wiley & Sons, Inc. (1992-2000), Chapter 17, Section 17.1. Related patents include US Patents 6,706,484; 6,297,004 and 5,571,698 and the like.

Detection of antibody binding may be performed by contacting the antibody-antigen complex with a second antibody linked to an enzyme, such as alkaline phosphatase or horseradish peroxidase, or a fluorescent marker, such as FITC or Cy3. Other enzymes or markers may be employed and are well known to one with skill in the art. Alternatively the antibody itself may be labeled for example, with a radioisotope or enzyme for *in vivo* or *in vitro* detection of the related glycans.

Two types of monoclonal antibodies are primarily used in cancer treatments:

*Naked* monoclonal antibodies are those without any drug or radioactive material attached to them. Naked antibodies attach themselves to specific antigens on cancer cells. They can be used in different ways, for example some mark the cancer cell for eradication by the immune system; while others such as Trastuzumab (Herceptin), attach to certain antigen sites thereby blocking the binding of ligands and preventing the cancer cell proliferation.

*Conjugated* monoclonal antibodies are those joined to a chemotherapy drug, radioactive particle, or a toxin. Conjugated monoclonal antibodies joined to drugs, toxins, or radioactive atoms are used as delivery vehicles to take those substances directly to the cancer cells. The MAb acts as a homing device, circulating in the body until it finds a cancer cell with a matching antigen. It delivers the toxic substance to where it is needed most, minimizing damage to normal cells in other parts of the body.

The construction and selection of human antibody fragments from combinatorial libraries displayed on filamentous phage surfaces has become the alternative to the laborious monoclonal techniques. Some libraries are generated by a random combination of variable light (VL) and variable heavy (VH) chain genes produced as antigen binding (Fab) or single chain variable (scFv) antibody fragments. Theoretically, each clone codes for a specific antigen-binding site, corresponding to a natural repertoire, increased by an artificial domain combination that extrapolates the individual repertoire. This panning procedure mimics the B-cell clonal selection system *in vitro* by specifically enriching phage particles that display antibodies with a desired specificity. Several human antibody combinatorial libraries displayed on filamentous phage surface have been built by various groups, from either naive (Griffiths et al., 1993; de Haard et al., 1999; Lu et al., 2002) or immunized/infected repertoires (Portolano et al., 1993; Wu et al., 2001); this system has been applied to select antibodies against different antigens, including melanoma (Cai and Garen, 1995), colorectal (Somers et al., 2002) and prostate (Mintz et al., 2003) cancer proteins.

In some application a PEGylated antibody or antibody fragment will be desired. PEGylation stabilizes and can increase the half-life in serum of a molecule.

### Peptide and Peptide Analogs

The present disclosure provides a diagnostic and or therapeutic agent which can be a peptide or a peptide analog. Screening a library of such molecules may identify the peptide or peptide analog. Alternatively, a peptide analog may be synthesized based on a peptide identified in a screen of a peptide library. The peptide analogs include linear and cyclic peptides and peptidomimetics. A peptide mimetic or peptidomimetic is a molecule that mimics the biological activity of a peptide but is not completely peptidic in nature. Whether completely or partially non-peptide, peptidomimetics provide a spatial arrangement of chemical moieties that closely resembles the three-dimensional arrangement of groups in the peptide on which the peptidomimetic is based. As a result of this similar active-site geometry, the peptidomimetic has effects on biological systems, which are similar to the biological activity of the peptide.

The present disclosure encompasses peptide and peptide analog compositions. Said peptide/peptidomimetic compositions are effective in situations where cancer cell death is desired.

There are clear advantages for using a mimetic of a given peptide rather than the peptide itself, because peptides commonly exhibit two undesirable properties: poor bioavailability and short duration of action. Peptide mimetics offer a route around these two major obstacles, since the molecules concerned are have a long duration of action. Small peptidomimetics of 3-6 amino acids exhibit improved patient compliance since they can be administered orally compared with parenteral administration for peptides or larger peptidomimetics. Furthermore there are problems associated with stability, storage and immunoreactivity for peptides that are not experienced with peptide mimetics.

One aspect of the present provides for a peptidomimetic or a peptide or peptide analog, which mimics the structural features of the critical minimal epitope. Binding of the peptidomimetic preferably induces the binding protein to carry out the normal function caused by such binding (agonist).

A primary goal in the design of peptide mimetics has been to reduce the susceptibility of mimics to cleavage and inactivation by serum or gastric peptidases. In one approach, one or more amide bonds have been replaced in an essentially isosteric manner by a variety of chemical functional groups. In another approach, a variety of uncoded or modified amino acids such as D-amino acids and N-methyl amino acids have been used to modify mammalian peptides.

Alternatively, a presumed bioactive conformation has been stabilized by a covalent modification, such as cyclization or by incorporation of γ-lactam or other types of bridges as disclosed for example in US Patent 5,811,392. In US Patent 5,552,534, non-peptide compounds are disclosed which mimic or inhibit the chemical and/or biological activity of a variety of peptides. Such compounds can be produced by appending to certain core species, such as the tetrahydropyranyl ring, chemical functional groups, which cause the compounds to be at least partially cross-reactive with the peptide. As will be recognized, compounds which mimic or inhibit peptides are to varying degrees cross-reactive therewith. Other techniques for preparing peptidomimetics are disclosed in US Patent 5,550,251 and US Patent 5,288,707, for example.

### Pharmaceutical compositions

The disclosure also provides a composition comprising the molecule of the disclosure. In one embodiment, the composition is a pharmaceutical composition. Pharmaceutical compositions may be manufactured by processes well known in the art, e.g., by means of conventional mixing, dissolving, granulating, grinding, pulverizing, dragee-making, levigating, emulsifying, encapsulating, entrapping or lyophilizing processes.

Pharmaceutical compositions for use in accordance with the present disclosure thus may be formulated in conventional manner using one or more physiologically acceptable carriers comprising excipients and auxiliaries, which facilitate processing of the active compounds into preparations which, can be used pharmaceutically. Proper formulation is dependent upon the route of administration chosen.

For injection, the compounds may be formulated in aqueous solutions, preferably in physiologically compatible buffers such as Hank's solution, Ringer's solution, or physiological saline buffer. For transmucosal administration, penetrants appropriate to the barrier to be permeated are used in the formulation. Such penetrants, for example polyethylene glycol, are generally known in the art. Pharmaceutical compositions which can be used orally, include push-fit capsules.

For administration by inhalation, the molecules for use according to the present disclosure are conveniently delivered in the form of an aerosol spray presentation from a pressurized pack or a nebulizer with the use of a suitable propellant, e.g., dichlorodifluoromethane, trichlorofluoromethane, dichloro-tetrafluoroethane or carbon dioxide. In the case of a pressurized aerosol, the dosage unit may be determined by providing a valve to deliver a metered amount. Capsules and cartridges of, e.g., gelatin for use in an inhaler or insufflator, may be formulated containing a powder mix of the polypeptide and a suitable powder base such as lactose or starch.

Pharmaceutical compositions for parenteral administration include aqueous solutions of the active ingredients in water-soluble form. Additionally, suspensions of the active compounds may be prepared as appropriate oily injection suspensions. Suitable natural or synthetic carriers are well known in the art (Pillai *et al*., 2001). Optionally, the suspension may also contain suitable stabilizers or agents, which increase the solubility of the compounds, to allow for the preparation of highly concentrated solutions. Alternatively, the active ingredient may be in powder form for reconstitution with a suitable vehicle, e.g., sterile pyrogen-free water, before use.

Herein the terms "excipient" and "carrier" refer to an inert substance added to a pharmaceutical composition to further facilitate administration of a compound. Examples, without limitation, of excipients include calcium carbonate, calcium phosphate, various sugars and types of starch, cellulose derivatives, gelatin, vegetable oils and polyethylene glycols. Pharmaceutical compositions may also include one or more additional active ingredients.

Pharmaceutical compositions suitable for use in context of the present disclosure include compositions wherein the active ingredients are contained in an amount effective to achieve the intended purpose. All formulations for administration should be in dosages suitable for the chosen route of administration. More specifically, a "therapeutically effective" dose means an amount of a compound effective to prevent, alleviate or ameliorate symptoms of a disease of the subject being treated. Determination of a therapeutically effective amount is well within the capability of those skilled in the art, especially in light of the detailed disclosure provided herein.

Suitable delivery reagents for administration in conjunction with the present antibody and peptide molecules include liposomes. Liposomes suitable for use in the disclosure are formed from standard vesicle-forming lipids, which generally include neutral or negatively charged phospholipids and a sterol, such as cholesterol. The selection of lipids is typically guided by consideration of factors such as the desired liposome size and half-life of the liposomes in the blood stream. In this case, the present antibodies will bind on the surface of activated liposomes, rather than being encapsulating the molecules of the present invention, and will enable presentation of the molecules of the present disclosure with multiple binding sites.

Toxicity and therapeutic efficacy of the compositions described herein can be determined by standard pharmaceutical procedures in cell cultures or experimental animals, e.g., by determining the IC₅₀ (the concentration which provides 50% inhibition) and the LD₅₀ (lethal dose causing death in 50% of the tested animals) for a subject compound. The data obtained from these cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. Depending on the severity and responsiveness of the condition to be treated, dosing can also be a single administration of a slow release composition, with course of treatment lasting from several days to several weeks or until cure is effected or diminution of the disease state is achieved. The amount of a composition to be administered will, of course, be dependent on the subject being treated, the severity of the affliction, the manner of administration, the judgment of the prescribing physician, and all other relevant factors.

### Methods of Treatment

The disclosure additionally provides a method of treating a subject in need thereof, with a molecule of the disclosure or with a composition that comprises said molecule as an active ingredient. The method of treatment comprises administering a molecule or composition of the disclosure to a subject. In one embodiment, the subject is a human. In another embodiment the disease to be prevented, treated or detected is cancer.

Accordingly the present disclosure relates to a method for the treatment of cancer in a subject in need thereof the method comprising the step of:
administering to the subject a pharmaceutical composition comprising a to a carbohydrate binding molecule selected from the group consisting of an antibody, a peptide and a small organic compound, wherein binding of the molecule to a tumor-associated carbohydrate antigen on a cancer cell induces death of the cancer cell, and wherein said molecule is able to displace a lectin, wherein binding of the lectin to the tumor-associated carbohydrate antigen on the cancer cell induces death of said cancer cell.

As used herein the terms "treating" or "treatment" should be interpreted in their broadest possible context. Accordingly, "treatment" broadly includes amelioration of the symptoms or severity of a particular disorder, for example a reduction in the rate of cell proliferation, reduction in the growth rate of a tumor, partial or full regression of a tumor, or preventing or otherwise reducing the risk of metastases or of developing further tumors.

As used herein, a "therapeutically effective amount", or an "effective amount" is an amount necessary to at least partly attain a desired response. A person of ordinary skill in the art will be able without undue experimentation to determine an effective amount of a compound of this invention for a given disease or tumor.

As used herein, the phrase "cancer" includes diseases and disorders in which timely growth arrest does not ensue and cells grow or proliferate without restraint, for example in malignant and benign neoplasias. Herein cancer relates to abnormal proliferative growth in of any one or more of the following organs and tissues: lung, bone, pancreatic, skin, head or neck, eye, uterus, ovary, rectum, anal region, stomach, colon, breast, fallopian tubes, endometrium, cervix, vagina, vulva, lymph including Hodgkin's and non-Hodgkin's and lymphocytic lymphomas, esophagus, small intestine, endocrine system, thyroid gland, parathyroid gland, adrenal gland, soft tissue, urethra, penis, prostate, blood including chronic or acute leukemia, bladder, kidney, central nervous system (CNS) including spinal axis tumors, brain stem glioma; pituitary.

The dose of the molecule to be administrated to a subject, in the context of the present disclosure should be sufficient to effect a beneficial therapeutic response in the subject over time, or to inhibit tumor growth, progression and or metastasis. Thus, the molecule may be administered to a subject in an amount sufficient to alleviate, reduce, cure or at least partially arrest the disease.

The dose will be determined by the activity of the therapeutic composition produced and the condition of the subject, as well as the body weight or surface area of the subject to be treated. The size of the dose also will be determined by the existence, nature, and extent of any adverse side effects that accompany the administration of a particular therapeutic composition in a particular subject. In determining the effective amount of the therapeutic composition to be administered, the physician needs to evaluate circulating plasma levels, toxicity, and progression of the disease.

### Lung Cancer

The two major categories of lung cancer are non-small cell lung cancer (NSCLC) and small cell lung cancer (SCLC). Less common cancers of the lung are known as carcinoids, cylindromas, and certain sarcomas. Cancers in the lung may have metastasized from other primary sites, such as the breast, thyroid, or colon.

### Non-Small Cell Lung Cancers

Non-small cell lung cancers are categorized into three types: squamous cell carcinoma (also called epidermoid carcinoma), adenocarcinoma, and large cell carcinoma. These separate types are grouped together because, in early stages before the cancers have spread, they all can be treated surgically.

Tumors formed from squamous cells are usually found in the center of the lung, either in a major lobe or in one of the main airway branches. They may grow to large sizes and form cavities in the lungs. When a squamous cell cancer metastasizes, it may reach the bone, adrenal glands, liver, small intestine, and brain. Squamous cell carcinoma is nearly always caused by smoking and used to be the most common cancer. It still makes up between 25% and 40% of all lung cancers.

Adenocarcinomas usually arise from the mucus-producing cells in the lung; about two-thirds of adenocarcinomas develop in the outer regions of the lung, while one-third develops centrally. NSCLC adenocarcinoma is the predominant lung cancer in women. Adenocarcinoma is usually a slow-growing cancer, but can be difficult to detect because the disease typically involves the periphery of the lung, resulting in fewer early symptoms than cancers that develop centrally, near the airways. When signs of the disease do occur, they may include painful breathing, shortness of breath, wheezing, and a persistent cough. Oftentimes, lung adenocarcinoma has already metastasized by the time any symptoms develop, resulting in an overall five year survival rate associated with the disease that is less than 20 percent. Secondary tumors most commonly form in the opposite lung, the brain, spinal cord, bones, liver, and adrenal glands. Additional symptoms related to tumor growth in these or other areas of the body sometimes develop before signs of the primary tumor.

Bronchoalveolar lung cancer is a subtype of adenocarcinoma. It develops as a layer of column-like cells on the lung and spreads through the airways, causing great volumes of sputum. This cancer also is increasing in incidence.

Large Cell Carcinoma. Large cell carcinoma, which makes up about 10% to 20% of lung cancers, includes cancers that cannot be identified under the microscope as squamous cell cancers or adenocarcinomas.

### Diagnostics and Imaging

The present disclosure further provides the molecules as described herein for the diagnosis and imaging of tumors. The compound of the present disclosure preferably has affinity for tumor cells but low or no affinity for normal tissue.

According to one embodiment the present disclosure provides a method for the diagnosis of cancer in a subject wherein the method comprises the steps of:
contacting a specimen comprising suspected cancer cells from the subject with a molecule comprising the antigen-binding portion of an antibody having a affinity for a cell membrane associated glycan wherein the glycan has the ability to bind to a lectin and the binding induces death of the cell; and
detecting whether the molecule binds to the specimen; wherein detection of binding between the specimen and the molecule provides a positive indication in the diagnosis cancer.

The method may be for the purpose of disease detection, establishing the prognostic course of the disease, for determining the success of various therapeutic regimes, or for establishing admission criteria of a specific patient to a specific therapeutic regime.

According to one embodiment a suitable specimen or sample from a subject is a bodily fluid or tissue sample from the subject, the subject having or suspected of having cancer. A suitable biological specimen includes, but is not limited to, colorectal tissue or cells, blood serum, lymph node tissue or cells, spleen, liver or lung tissue or cells, ascitic fluid obtained from the abdominal cavity, fecal material and fluid or phlegm obtained from the lung. In one embodiment, the suitable biological specimen is lung tissue. In another embodiment the suitable biological specimen is colorectal tissue. Alternatively, the biological specimen may be cells or tissue isolated from the subject that have been cultured in cell culture. Methods of obtaining a suitable biological specimen from a subject are known to those skilled in art.

The term "imaging agent" denotes a label compound that can be visualized with imaging equipment. Such label can be radioactive, fluorescent, calorimetric, or magnetic. The imaging agent may be selected from a metallic or non-metallic imaging agent. The "imaging agent carrier" is preferably a metal chelator that is able to bind radiometals useful in cancer imaging.

The term "metal chelator" refers to a chemical species (molecule, compound) having at least one coordinating group which is able to complex (coordinate) with a metal ion. A chelator may be selected from ethylenediamine, propylenediamine, diethylenetriamine, triethylenetetraamine, ethylenediaminetetraaceto, oxalato, hydroxyquinolates, hydroxyqinones, aminoquinones, dipyridyl, phenanthroline, acetylacetone, oxalic acid and bifunctional acids.

Preferable chelators that can bind radioactive molecules useful in cancer imaging are DOTA (1,4,7,10-tetraazacyclododecan-1,4,7,10-tetraacetic acid) and DTPA (diethylene-triaminopentaacetic acid).

### Lectins

Identification of glycoprotein glycoforms is becoming an increasingly important laboratory contribution to the diagnosis and management of human diseases as more diseases are found to result from glycan structural alterations.

To date numerous carbohydrate (glycan) binding proteins (CBP; GBP) have been identified, demonstrated or implicated in mediating various cellular events through protein-carbohydrate interactions. Protein-carbohydrate interactions have been shown to mediate a variety of biological activities such as homeostasis and immune responses. Immune activities include pathogen recognition and neutralization, leukocyte trafficking, phagocytosis, antigen uptake and processing, and apoptosis. Lectins that participate in such reactions include but are not limited to the following groups:

Galectins are a rapidly growing family of animal lectins. All of them share galactose-specificity. Ca-dependent (C-type) animal lectins form an extremely large family, composed of members having diverse structures and functions. Among this C-type lectin family, selectins form a distinguished subfamily by their specific function in leukocyte adhesion to endothelial cells through sialyl-LewisX recognition. Collectins, another subfamily of C-type lectins specific for mannose, have a unique structure consisting of a C-type lectin domain and a collagen-like domain, are believed to be involved in innate immunity.

On the other hand, invertebrates are known to contain various lectins in their body fluids, probably as body-protection factors.

The legume lectin family consists of a large number of members, such as ConA, with variable saccharide specificity comparable to C-type lectins. Recently, mechanistic studies have been made on how such specificities and affinities are controlled.

Ricin was the first lectin investigated in Russia more than 100 years ago. It is now evident that ricin has many other homologous members which differ in either toxicity or sugar-binding specificities. Ricin is actually the enzyme RNA-N-glycosidase.

According to one embodiment the present disclosure provides a method for the diagnosis of cancer in a subject wherein the method comprises the steps of:
a. contacting a specimen comprising suspected cancer cells from the subject with a lectin selected from the group consisting of PNA, DBA, UEA, SBA, LTL, VVA, MAA, and PHA; and
b. detecting whether the lectin binds to the specimen;
wherein detection of binding between the specimen and the lectin provides a positive indication in the diagnosis of a cancer.

The following examples are presented in order to more fully illustrate some embodiments of the invention. They should, in no way be construed, however, as limiting the broad scope of the invention. One skilled in the art can readily devise many variations and modifications of the principles disclosed herein without departing from the scope of the invention.

### EXAMPLES

Carbohydrate residues of the membrane associated glycoproteins and glycolipids can be detected using lectins. Thus, the use of lectins to discover specific carbohydrates present on the cell surface of a diseased cell can be applied to identify new cancer markers. Several lectins that bind selectively to human non-small cell lung cancer (NSCLC) and induce their cell death have been identified.

Abbreviations: FCS: Fetal calf serum; DMEM: Dulbecco's modified Eagle's medium; PBS: Phosphate buffered saline; BSA: bovine serum albumin; HRP: horse radish peroxidase; DAPI: 4',6'-diamidino-2-phenylindole (DNA stain); PNA: Peanut (*Arachis hyposaea*) Agglutinin; DBA: *Dolichos biflorus* Agglutinin; UEA: *Ulex europeaus* Agglutinin; SBA: soybean (*Glycine max*) Agglutinin ; RCA: *Ricin communis* Agglutinin; PHA-L: *Phaseolus vulgaris-L* Agglutinin; WGA: Wheat germ (*triticum vulgaris*) Agglutinin; MAA: *Maackia amurensis* Agglutinin; LICA: Ligand induced cell apoptosis agent; RT: room temperature;

### Methods:

### Tissue Culture

Human colon cancer cell lines HT-29, SW480, HCT116, Colo320; human pancreatic cancer cells lines CoLo357, Panc1, MiaPaca, Human Non Small Cell Lung Carcinoma cell lines A549 and H1299 and normal skin fibroblasts were grown and maintained in DMEM supplemented with 10% FCS, penicillin and streptomycin at 37°C, in an atmosphere of 95% oxygen and 5% CO₂.

Cell Viability Assay was performed on cancer cell lines. Cells were seeded in 96 well plates (5x10³ cells/well) in 10% FCS growing DMEM media. Cells were treated with lectins or antibodies to an antibody to a glycosylated molecule (SN3). SN3 is a monoclonal antibody directed to the sialic acid moiety of CD24 (Fukokawa et al, 1986). Cells were also incubated with human serum of different individuals at different dilutions (1:500, 1:100, 1:50, 1:25) for different time intervals (48 hours, 96 hours and 144 hours). At the end of the experiment, cell viability was determined by methylene blue assay.

### Methylene Blue Assay

The methylene blue (MB) assay was used to assay cell growth and viability including cell proliferation and doubling time, cell inhibition and apoptosis and to calibrate numbers of cells seeded on plate for the ELISA test. Cells seeded in 96 well plates were washed 3 times with PBS after which the cells are fixed with 200 µl 4% formaldehyde solution for 2 hrs at room temperature. Plates were washed with PBS and then incubated with 150 µl RNAse A (3 ug/ml) for 30 min at room temperature. Plates were equilibrated with 200 µl 0.1M sodium borate buffer pH 8.5, stained with 200us 0.5% methylene blue (0.1 M sodium borate buffer ph 8.5) and then incubated for 10 min at room temperature. The cells were washed with tap water and cell-bound dye was eluted with 200 ul 0.1M HCl. Eluted methylene blue was read in an ELISA plate reader at 595 nm. Methylene blue binds DNA in basic solution, and it is extracted in acidic condition. Staining of DNA by Methylene Blue correlates with cell survival.

### Cell surface protein Enzyme Linked Immuno-Sorbent Assay (ELISA)

Serum for ELISA was prepared from 2.5 ml of venous blood that was withdrawn from each subject, left to clot at room temperature for 30 min and then centrifuged at 1500 g for 15 min. Serum was separated into sterile aliquots and was stored at -80oC. Part of the serum is depleted of antibodies, and in some reaction condition 0.1 mM EDTA is added to the reaction. Cells were grown to confluence of 90% in 96-well plates. Cells are fixed with 4% formaldehyde for 2 hours and than washed with PBS. Blocking solution (100ul 1% BSA in PBS) was added to each well and plates were incubated at room temperature for one hour. Mouse antibodies, normal sera, and sera from affected individuals were diluted in 50 mM Tris-Cl (pH 8.0), 0.15 M NaCl, and 1 mM EDTA containing 1% BSA. Cells were first incubated with serum or anti-carbohydrate antibodies for 2 hours at room temperature. Cells were washed with PBS, and than incubate again with SN3 or serum respectively to generate competitive reaction. After washing with PBS, cells were incubated with horseradish peroxidase-labeled anti mouse or anti human antibodies (Vector Laboratories Inc., Burlingame, CA) at room temperature for 30 min. TMB substrate (perborate 3,3',5,5'-tetramethylbenzidine; 100 µl) reacts with HRP to create a blue colored solution. Sensitivity is enhanced by addition of 100 µl sulfuric acid (0.3M) stop solution, turning the solution color yellow. The reactions were determined by measuring solution absorbance at 450 nm. The ELISA assay results were correlated and adjusted to cell number as measured by methylene blue assay.

### Therapeutic potential of lectin and anti-carbohydrate antibodies

Cells were seeded in a 96 well plate (2x10⁴ cells/well). Following 24 hours, cells were treated with different lectins for 6 hours in serum free media. Medium was changed to DMEM containing 10% serum and cells were grown for additional 96 hours. Cell viability was analyzed by methylene blue assay.

### Lectin immunohistochemical staining of tissue sections

Paraffin fixed section of about 10-20 µm of tumors from affected individuals were obtained from the pathology department at a local hospital.

The sections were deparaffinized and hydrated using the following washes: xylene (15 min): xylene (15 min): 100% alcohol (5min): 100% alcohol (5min): 95% alcohol (5 min): 75% alcohol (5 min): Washing in PBS 2 x 5min: PBS was added to cover sections, and left for 5 min (if processing several sections, immerse in PBS in coping jar). During the PBS rinse, blocking solution of 0.5mg/ml BSA in PBS (need ∼500-1000 ul per slide) was prepared. PBS was removed blocking solution was added to cover the sections, which were incubated for 30 min at RT.

Biotinylated conjugates of PNA, DBA, UEA, SBA and RCA were used to detect the expression of carbohydrate moieties in glycoconjugates of NSCLC specimens from diagnosed individuals. The staining of normal tissue within the sections was monitored to determine the binding specificity. Lectin histology was performed on sections from paraffin embedded specimens of donor individuals. Preparation of biotinylated lectin solution (for each lectin DBA, PNA, UEA, SBA & RCA) (∼300-500ul per slide): 1/500 (0.01ug/ul) dilution of lectin stock in PBS.

The blocking solution was removed and lectin solution was added to each slide to cover sections. Slides were incubated for 20 min at RT followed by rinses (3 x 10min) with PBS. Streptavidin Cy3 and avidin Fluorescein solutions (1:500 dilution (1ng/ul)) were added to slides and sections were incubated for 20 min at RT, followed by PBS rinses (3x10min). The slides were DAPI stained and mounted in Gel Mount media according to standard procedure.

### Hoechst 33258 staining

Cells were exposed to 100 µg/ml lectin samples for 48 h, washed with PBS and fixed in fresh Carnoy's fixative solution (methanol/acetic acid, 3:1 by vol.). After staining with Hoechst 33258 (0.5 µg/ml in PBS) for 30 min, representative photomicrographs were taken under UV illumination (380 nm). Apoptosis can also be determined using commercially available kits.

### TUNEL assay

TUNEL (Terminal deoxynucleotidyl transferase Biotin-dUTP Nick End Labeling) tests of paraffin slides were performed using a commercial kit (i.e. Upstate Biotechnology), following the manufacturer's instructions. The slides were counterstained with hematoxylin and viewed under a light microscope.

### Apoptosis and DNA fragmentation analysis

Cells undergoing apoptosis, degrade their chromatin DNA, which can be analyzed by DNA purification and gel electrophoresis.

A cell suspension is precipitated at 200xg at 4°C for 10 min. A solution of lysis buffer is added to the pellet and the tube vortexed vigorously. Following cell lysis and disruption of the nuclear structure the fragmented DNA is separated from intact chromatin, the mixture is centrifuged. The supernatant is carefully transferred into a new tube and treated with proteinase K (10mg/ml). The nucleic acid is precipitated and recovered by centrifugation The DNA can analyzed by agarose gel electrophoresis.

### Annexin V assay

In normal cells, phosphatidylserine (PS) residues are found in the inner membrane of the cytoplasmic membrane. During apoptosis, the PS residues are translocated and are externalized. In general, though not always, this is an early event in apoptosis and is thought to be a signal to neighboring cells that a cell is ready to be phagocytosed. Annexin-V is a specific PS-binding protein that can be used to detect apoptotic cells and is commercially available conjugated to different fluorochromes.

Cells are washed and put on ice. An Annexin V solution is added, and cells are incubated on ice. Cells are then washed and analyzed by flow cytometry (FACS). Early apoptotic cells are Annexin V positive.

### Western blot analysis of blood serum

Serum proteins were treated with Sepharose^{®} bound mixed lectins PHA-L, PNA, DBA, WGA, UEA, SBA and RCA for 1 hour. Sepharose complexes were precipitated and washed with PBS, than analyzed on a gradient (6-12%) sodium dodecyl sulphate polyacrylamide gel electrophoresis (SDS-PAGE) and detected by immunoblotting. Proteins were transferred to nitrocellulose membranes. Filters were saturated for 1 h with non-fat dry milk (3% w/v) in Tris buffered saline (TBS) and subsequently incubated with a Muc1 antibodies in TBS supplemented with 1% non-fat dry milk for 1 h under continuous stirring at room temperature. After exhaustive rinsing with TBS/0.5% Tween-20, membranes were incubated with HRP-conjugated goat anti-mouse IgG (Sigma) or streptavidin HRP in TBS supplemented with 1% non-fat dry milk for 1 h. After washing with TBS containing 0.5% Tween-20, the protein bands were detected using a chemiluminescence system. Prestained molecular weight markers (Sigma) were used as standards.

### Analysis of blood samples for binding to lectins

Sepharose^{®} bound lectins were analyzed for interactions with serum proteins. Human serum from healthy individuals and cancer patients are analyzed for proteins that bind the different lectins. Glycoproteins that are captured by binding to lectins are detected by western blot analysis using fluorescent labeled probes such as antibodies to unique tumor-associated carbohydrate antigens and to lectins. In the present study antibodies that recognize Muc 1 glycoprotein were used.

A mix of lectins (PHA, PNA, DBA, RCA, WGA, SBA and UEA) was used for the identification of Muc1 cancer-associated protein in the serum.

### In vivo therapeutic potential of lectins and anti-carbohydrate antibodies

Subcutaneous tumors were produced by injecting (about 1.0x10⁴) of viable cancer cells into two flanks of each CD1 nude mice. The animals were palpated twice weekly to detect the presence and location of subcutaneous tumors. The time of appearance of the first tumor (latency period) and its size were recorded. Lectins and anti-carbohydrate SN3 antibodies were injected locally at the site of the tumor five days after the injection of cancer cells. The experiment ended when the untreated animal (control) developed tumors larger than 1.5 cm (about 4 weeks). At the termination of the experiment, half of the animals from each group were sacrificed following sodium pentothal anesthesia and cervical dislocation, while the other half will be evaluated for another 4 weeks to follow tumor development and metastasis with no additional antibody treatment. Necropsy included gross examination of the internal organs, including the lungs, heart, stomach, spleen, kidneys and liver. Each tumor diameter was measured using a micrometer caliper, and tumor weight was measured. Both the number of mice with tumors (incidence) and the volume of tumors/mice (tumor burden) were measured. At the termination of the experiment, animals were sacrificed following sodium pentothal anesthesia and cervical dislocation.

### Lectin displacement assay utilizing antibodies, antibody fragments, peptides or small molecules:

Lectins are used as probes for example by labeling with a fluorescent marker, to select for their displacement by candidate molecules using the following protocol:
1. Human cancer cells were seeded in 96 well plates in triplicates, and allowed to proliferate to about 70% confluence.
2. Cells were fixed with formaldehyde and washed with PBS.
3. Plates were sealed and stored at 4°C until used.
4. Each well was washed with 200 µl PBS / 1% Tween solution.
5. Non-specific binding was blocked by adding 100 µl of 1% BSA solution (1% (w/v) in 100mM NaHCO₃ pH-8.5) to each well and plate resealed. Plate was incubated at room temp for 1h.
6. Solution was aspirated from each well and wells were washed 3 times with 200 µl PBS / 1% Tween solution.
7. Labeled lectin in PBS was added to each well, incubated at 37°C.
8. Wells were washed 3 times with 200 µl PBS / 0.1% Tween solution.
9. 100 µl of phage library solution / peptide library solution / small compound library in PBS / 0.1 % Tween was added to each well, incubated for 1h at room temp.
10. Supernatant was collected and bound molecules isolated.

Alternatively, the lectin can be added following addition of the library, for example at step 7 the candidate molecules (library) are added and at step 9 the lectin is added. Under those circumstances, displacement of the candidate molecule will be confirmed by visualizing labeled lectin on the cell surface and candidate molecule can be isolated from supernatant. When using phage library, the phage host can be inoculated in order to propagate isolated phage.

### Analysis of blood samples for binding to lectins

Lectin affinity separation is based on the ability of lectins to bind specifically to oligosaccharide structures on glycoconjugates. This technique is highly selective and thus provides a high purity target compound whilst the biological activity and natural glycosylation pattern is conserved due to the mild separation parameters.

A series of Sepharose^{®} bound lectins will be analyzed for interactions with serum proteins. Each lectin can bind several different proteins, depending on the surface glycan. Human serum from healthy individuals and cancer patients are analyzed for proteins that bind the different lectins. Glycoproteins that are captured by binding to lectins are detected by western blot analysis using fluorescent labeled probes such as antibodies to unique tumor-associated carbohydrate antigens and to lectins. The labeled probes can be antibodies that recognize the glycoprotein (i.e. anti-Muc1, CD14, CD16, CD59, CD79, CD87, CD30, CD44, CD43, CD147, CD146, CD63, CD22, CD29, CD33, CD34, PSA, B7-H4, Thomsen-Friedenreich antigen, CD7, ErbB3, EGF Receptor, IGF1 Receptor, gp130 - Muc1 and Muc 18, L-CanAg, a mucin-like glycoprotein, CEA and the like) or other probes such as lectins.

The glycoproteins are detected by micro-sequencing and MALDI. In such way, the pattern of glycoproteins in body fluids will be mapped for healthy and affected individuals. Examples of lectins that will be used for the identification of cancer-associated proteins can be seen in Tables 6 and 7, *infra.*

The process of lectin affinity separation is shown in figure 1. The method of lectin affinity separation comprises the steps of:
a. providing an affinity column to which at least one lectin is immobilized;
b. applying to the affinity column a specimen from a subject comprising target cell-associated molecules, wherein said cell-associated molecules are complementary to the binding site of the immobilized lectin;
c. washing the column under conditions wherein the cell-associated molecules specifically bind to the lectin;
d. applying an elution buffer and collecting purified and concentrated cell-associated molecules in the eluate; and
e. analyzing said cell-associated molecules.

The elution buffer may elute the cell-associated molecules according to pH, competitive molecules or other methods known in the art.

### Results:

### Staining of cells and apoptosis induction using antibodies to sialic acid of glycoproteins.

Three monoclonal antibodies (mAbs), termed SN3, SN3a, and SN3b, which are directed to sialic acid of a glycoprotein(s) on human non-T leukemia cells have been characterized (Fukukawa et al., 1986). These mAbs were generated by immunizing mice with an antigen preparation isolated from cell-membrane glycoconjugates of NALM-1, a pre-B leukemia cell line. In a test using SN3 and SN3b with uncultured cell specimens derived from various cancer patients, the mAbs primarily reacted with non-T/non-B and B HLL specimens, as well as with chronic myelocytic leukemia specimens. The biochemical nature of antigenic determinants defined by the three mAbs was studied by treating the non-T leukemia cells with sialidase and proteases. The results, shown in figure 2 show that the antigenic determinants defined by these mAbs all contain a sialic acid residue(s) that is attached to the cells via a protein backbone(s).

Figure 2 shows the results of staining cancer cell lines with the SN3 monoclonal antibody. Human colorectal cancer (CRC) cell lines (HT29, CoLo320, SW480, HCT116, CaCo2), normal rat colon IEC 18 cells, and human pancreatic tumor cells (Panc 1 and Colo357) were analyzed for cell surface sialic acid residue staining using ELISA assay and SN3 antibodies. The staining was normalized with cell number, using methylene blue assay. The HT29 and Colo357 cells exhibited the strongest staining. However, SW480, HCT116 and Panel develop tumors in CD1 nude mice, much faster than when HT29 and Colo357 are injected subcutaneous to CD1 mice.

Inhibition of cell proliferation is mediated by SN3 antibodies. Human colon cancer cell lines HT29, SW480 and HCT116 and pancreatic cancer cell viability was measured by methylene blue assay following exposure to SN3 monoclonal antibodies during various incubation periods. Rat IEC18 are normal colon cells.

Figure 3: Cell viability was tested with varying doses of SN3 antibody. The assay was performed in 96 well plates and cells were tested for viability using methylene blue assay 96 hours later. All experiments were done in triplicate, to obtain highest efficacy possible. As demonstrated in figure 3, growth inhibition is observed in a dose-dependent manner. There are cells that are more sensitive to SN3 antibodies.

The human CRC HT29 cell line and Colo357 human pancreatic cancer cells are highly stained with SN3, and SN3 inhibits their growth dramatically. Even low levels of SN3 staining as seen in Panel and HCT116 cancer calls, enable their growth inhibition by SN3 antibodies.

### Serum components compete with the binding of SN3 antibodies on HT29 cells.

SN3 stains and inhibits cell proliferation of HT29 CRC cells (figures 2 and 3). Without wishing to be bound to theory, there are serum components that bind to the cell surface associated carbohydrate moieties, and transfer the same functional activity as SN3 does. Therefore, either antibodies to those moieties or lectins that bind to sugar moieties may be found in the serum of normal individuals, and their function is to recognize these tagged sequences and induce death of cells that bear these sequences.

An ELISA analysis was performed on HT29 cells. Cells were seeded in 96 well plates (1x10⁵ cells/well) and 24 hours later cells were incubated with human sera. SN3 antibody was used as second binder to analyze displacement of serum component by the antibody. The binding of SN3 antibodies was visualized by anti-mouse HRP antibodies, and human immunoglobulins were analyzed by anti-human HRP antibodies.

Displacement of human antibodies that recognize the same epitope as SN3 can be calculated when it is standardized to serum binding without the addition of SN3 antibodies, as is shown in table 1, hereinbelow.

Displacement by SN3 antibodies differs from serum to serum. The numbers in parentheses in table 1 refer to the age of the individual (27, 23, 50, 65), the serum number (10, 15, 2840,16, 23,14) and its dilution (1:25). The values in the table represent absorbance (A₅₉₅) of HRP.

**Table 1: HT29 cell staining: displacement of serum Ig by SN3 antibody.**

| **2nd Binder** | **No binding** | **SN3** | | **No binding** | **SN3** | **3rd Binder** |
|---|---|---|---|---|---|---|
| **1^{st} Binder** | | | **1^{st} Binder** | | | |
| Normal serum (10 -1:25) | 0.05 | 1.176 | Normal serum (16 -1:25) | 0.041 | 1.256 | anti mouse HRP |
| Normal serum (10 -1:25) | 2.763 | 2.574 | Normal serum (16 -1:25) | 2.541 | 2.221 | anti human HRP |
| Normal serum (15 -1:25) | 0.046 | 1.162 | Normal serum (14 -1:25) | 0.059 | 0.015 | anti mouse HRP |
| Normal serum (15 -1:25) | 2.754 | 2.698 | Normal serum (14 -1:25) | 2.745 | 2.412 | anti human HRP |
| | No binding | Normal serum (10-1:25) | Normal serum (15-1:25) | Normal serum (16-1:25) | Normal serum (14 -1:25) | |
| SN3 | 1.125 | 1.239 | 1.081 | 1.186 | 1.154 | anti mouseHRP |
| SN3 | 0.255 | 2.027 | 2.074 | 1.668 | 1.958 | anti human HRP |
| Nothing | 0.075 | | | | | anti mouse HRP |
| Nothing | 0.065 | | | | | anti human HRP |

Different runs of the experiment revealed a potential pattern of displacement. Detection of human antibodies is done by using anti human HRP antibodies. HT29 CRC cells are plated on 96 well plates. Cells were incubated with diluted serum as indicated in the table. After washing extensively, SN3 mouse antibodies were used to displaced human antibodies, as detected by anti human HRP as a third binder. The serum and the antibodies should be diluted to the maximum to enhance specificity of displacement.

### The potential use of lectins in detecting serum cancer markers

Cancer cells are tagged with specific carbohydrate moieties and the above results show that human serum contains molecules that detect these moieties. The serum molecules can be, for example, antibodies to glycosylation moieties or specific human lectins that interact with these moieties on cancer cells. Cancer cells may secrete such tagged molecule into the serum, thus quenching lectins and antibodies. For diagnostic purposes it is important to quantitate the antibody and lectin levels as well as secreted cancer biomarkers in the serum.

Using a mixture of lectins (PHA, PNA, DBA, WGA, UEA, SBA and RCA) a Muc1 cancer biomarker was identified in human serum of breast cancer patients. Sera prepared from normal females (age >50 years) females diagnosed with breast cancer and females having recurring breast cancer were analyzed for the presence of Muc 1 in their serum as detected by binding of the lectin mixture.

Sera were incubated with a mix of Sepharose® bound lectins. Sepharose® complexes were eluted and analyzed by PAGE SDS protein gels. Muc 1 was detected by Western blot analysis using anti-Muc 1 antibodies (figure 4). The assay was done with individual lectins to conclude potential use of lectins as a tool to identify serum markers.

### Cell death induced by lectins

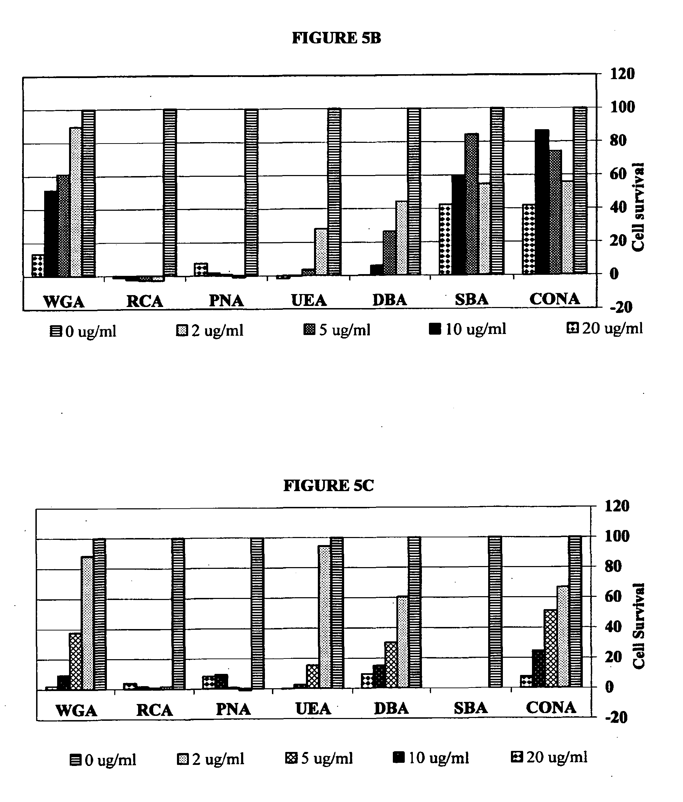

Several lectins were analyzed for their binding to cancer cells and death inducing activity. Skin fibroblasts from healthy individuals and human non-small cell lung cancer cell lines H1299 and A549 were used in this study. Cells were seeded for 24 hours in complete medium. Cells were treated with different lectins for 6 hours in serum free media, medium was changed to serum containing DMEM, and following 96 hours, cell viability was monitored by methylene blue assay. Normal epithelial cells from three individuals, and two NSCLC cell lines were analyzed. The experiments were performed in triplicate. Figures 5A-5C are representative of several experiments and demonstrate the differential effect of lectins on normal and cancer cells. Figure 5A shows the effects of certain lectins on normal cells. Figures 5B and 5C show the effect of the lectins on H1299 NSCLC cells, and A549 cells, respectively.

### Glycosylation moieties as recognition tags for cellular death signal

The results obtained so far suggest that monoclonal antibodies can recognize glycosylation moieties on cancer cells and deliver death signals to eliminate cancer cells. Without wishing to be bound to theory, antibodies found in the serum may have the same function and activities, suggesting a potential mechanism whereby glycosylation moieties as tags on cancer cells are recognized for elimination by the body.

These findings have opened a new avenue for developing tools to mimic this mechanism, and to identify potential human antibodies to glycosylation forms on cancer cells for sensing and elimination of cancer cells. These antibodies will have a great impact on cancer therapy, diagnosis and imaging.

### Methylene Blue assay

Figures 6A and 6B shows the results of lectin induced cell death as determined by the methylene blue assay. The wide side of the arrow represents the higher concentration of lectin. The mechanism of the assay is based on the binding of methylene blue to DNA.

### Animal studies

The *in vivo* studies aim to validate the potential of lectins to inhibit human non-small cell lung cancer (NSCLC) in an animal model. Human NSCLC (H1299 and A549) cancer cells were injected (1x10⁶ and 5x10⁶ respectively) into the flanks of CD1 nude mice. In preliminary experiments, we had determined the growth rate of tumors following injection of the NSCLC cancer cell lines. When the tumor reached a size of 50 mm³ (approximately 5 days) a mix of lectins (PNA, UEA and DBA) were injected into the tumor. Table 2 shows the results obtained with the animal groups, the injected cells and lectins, and their dosage:

**Table 2 : CD1 mice groups for dose A (1mg/Kg/injection x 5)**

| Cells | No cells | H1299 | A549 |
|---|---|---|---|
| Injection (intra tumor) | | 1x10⁶ | 5x10⁶ |
| PBS | 2 | 4 | 4 |
| Mix of lectins (PNA, DBA, UEA) | 2 | 4 | 4 |

It is believed that non-human lectins are not effective therapeutic compounds to treat cancers since they are immunogenic and human serum contains antibodies to some of those lectins. Therefore, the PNA, DBA, UEA, LTL, VVA, MAA, PHAA lectins will be used in screens to identify human antibodies or antibody fragment, peptides and small molecules that compete with binding and death activity with identified lectins. These products will then serve as therapeutic, diagnostic and imaging tools. Figures 7A-7F show the results of the mouse experiment. Figures 7A and 7B show two mice following subcutaneous injection of H 1299 cells. Note the large tumors that have formed on their fore flanks. Figures 7C-D show mice, which were injected with H1299 cells followed by treatment with a mix of lectins. Figures 7E and 7F are control mice injected with mix lectins only. These results demonstrate that lectins inhibit tumor growth, in vivo.

The above series of experiments have confirmed that plant lectins selectively bind to, and can induce death, of cancer cells (Higuchi, 2003; Kim et al., 1993; Schwarz et al., 1999). The next step is to perform a screening assay to identify novel therapeutic molecules that are able to mimic the function of the lectins. The first screen utilizes a human antibody phage display library to select for Fab fragment molecules that displace active lectins using assays we have developed.

### Screening_for selective killing lectins for NSCLC

Twelve different lectins were selected for selective staining and killing of NSCLC. Three different lectins PNA, DBA and UEA were identified. It has been shown previously that additional lectins bind selectively to lung cancer, however, death activity was not studied. The lectins VAA from *Viscum album* origin, HPA from the snail *Helix pomatia* origin and PVL from *Phaseolus vulgaris* origin, were found to stain lung cancer of endothelial origin. The assay is performed on human NSCLC cell lines H1299 and A549, using methylene blue assay.

### Validation of lectin activity in the presences of competitors

PNA, DBA and UEA induce selective killing of NSCLC cells while RCA is toxic to normal cells as well. If a lectin induces cell death in a ligand dependent manner, than using a blocking sugar should inhibit the cell death. Table 3 shows the specificity in staining and inducing cell death by several of the lectins tested. +: weak; +++++++: complete apoptosis

**Table 3:**

| Lectin | Blocking Sugar | A549 staining | A549 induce death |
|---|---|---|---|
| Con-A | Mannose | ++ | + |
| DBA | N-Acetylgalactose | + | +++ |
| PNA | Galactose | +++ | +++++ |
| RCA | Galactose | ++ | +++++++ |
| SBA | N-Acetylgalactose | +++ | ++ |
| UEA-1 | Fucose | +++ | +++++ |
| WGA | N-Acetylgalactose | ++ | ++ |

In parallel, experiments will be conducted in which A549 cells will be tested for death inducing activity by PNA, DBA and UEA in the presence of their specific blocking sugar (galactose, N-acetylgalactose and fucose, respectively). In order to corroborate the hypothesis that glycosylation moieties on cancer cells are used by components in the serum such as immunoglobulin for sensing and eliminating cancer cells, a displacement assay of selected lectins was performed with serum from normal individuals, and assayed by ELISA using HRP (horsed radish peroxidase) labeled anti-human antibodies. The results of such experiments infer the likelihood of finding such antibody fragment in the human phage display antibodies to be screened. This analysis will be done also in the presence of blocking sugars.

### Lectin staining of tissue samples

Galactose 1-3, N-acetyl galactosamine is the main binding moiety for the peanut agglutinin (PNA). Common nomenclature: PNA, GNL, MNL, PRA-I, PRA-II. Specificity: ß-Gal(1-3)GalNAc; *Dolichos biflorus* (DBA) a legume lectin with specificity to GalNAc; *Ulex europaeus* (UEA) a fucose binding lectin with specificity to Fucal-2Gal-R; *Glycine max* (SBA) has higher specificity for terminal α,βGalNAc than for α,βGal; *Ricinus communis* agglutinin-I (RCA-I) has specificity to Gal(β1->4)Glc

Figures 8-11 show staining of human tissue sections with the various lectins. Table 4 summarizes the results of the tissue staining with the lectins.

Tumor specimens from NSCLC diagnosed individuals were stained with the selected lectins, and the staining is undetectable in the surrounding normal tissue. NCSLC adenocarcinoma shows the highest specificity for the lectins, and PNA in particular.

**Table 4:**

| **Case #** | **NSCLC Cancer type** | **PNA** | **EUA** | **SBA** | **DBA** |
|---|---|---|---|---|---|
| 2420 02 2 | Adenocarcinoma | No staining | No staining | **Very selective** | No staining |
| 17464 00 1 (Figures 8A-8B) | Adenocarcinoma | **Very selective** | **Some selectivity** | No staining | No staining |
| 16968002 | Poorly differentiated Adenocarcinoma | No staining | No staining | No straining | No staining |
| 14441 00 5 (Figures 11A-11B) | Adenocarcinoma | **Very selective** | **Very selective** | **Very selective** | No staining |
| 9438 00 1 | Squamacarcinoma | No staining | **No staining** | No staining | No staining |
| 5765 00 4 (Figures 10A-10B) | Adenocarcinoma | **Very selective** | **some selectivity** | **Very selective** | No staining |
| 4710 00 2 | Adenocarcinoma | No staining | **very selective** | No staining | No staining |
| 478002 (Figures 9A-9B) | Adenocarcinoma | **Very selective** | **Very selective** | **Very selective** | **Very selective** |
| 10669 01 | Poorly differentiated Adenocarcinoma | No staining | No staining | No staining | No staining |
| 11863 01 | Moderate Adenocarcinoma | **Very selective** | No staining | No staining | No staining |
| 4756 01 | Metastatic Adenocarcinoma | No staining | **Very selective** | No staining | No staining |
| 5964 02 | Large Cell Carcinoma | No staining | No staining | No staining | No staining |
| 816500 | Large Cell Carcinoma | **Large area staining** | **Large area staining** | **Large area staining** | No staining |
| 2402302 | Large Cell Carcinoma | No staining | No staining | No staining | **Large area staining** |
| 18414 99 3 | Poorly differentiated adenocarcinoma | No staining | No staining | No staining | No staining |

The results show that PNA stains very specifically 5 of 8 cases (62.5%) of adenocarcinoma of the lung, EUA stains very specifically 4 of 8 cases (50%) of adenocarcinoma of the lung, SBA stains very specifically 4 of 8 cases (50%) of adenocarcinoma of the lung, and DBA stains very specifically 1 of 8 cases (12.5%) of adenocarcinoma of the lung. Thus, All adenocarcinoma of the non-small cell lung cancer cases are stained with at least one of the lectins very specifically. These findings show that lectins stain tumor tissue and differentiate between tumor and normal tissue especially in the case of adenocarcinoma.

These results, together with the discoveries that 1: death induced activity by lectins is ligand dependent, and is inhibited by competing ligands and 2: that components from normal serum (antibodies) compete with binding and apoptosis activity with selected lectins, provide support for the development of an assay to displace the lectin from its binding site by a potential ligand competitor, such as a phage that displays an antibody fragment. Isolation of high specificity competing LICAs on phages

A unique antibody phage display screening assay utilizing a lectin displacement assay, was developed to identify human antibody scFv fragment molecules that displace the active lectins, stain selectively cancer cells and induced cancer cell death. Affinity selection of a human scFv library (Azriel-Rosenfeld et al., 2004) was performed in parallel on fixed and live cancer cells. In a test sample, A546 cells are grown. Cells are grown on two 96 well plates up to 70% confluence. One plate was fixed and both plates were screened for phages that are displaced by labeled lectins to the supernatant. Eluted displaced phages were re-amplified and subjected to four or five consecutive rounds of panning with increasing washing stringency to select individual phages. Phagemid DNA, isolated after the last round of panning, will be analyzed and recloned into plasmids producing soluble Fab. The scFv fragments were tagged with maltose binding protein sequence for purification purposes and for identification in biological assays.

### Competitive analysis of isolated phages

Isolated phages (10⁹ transducing units) are incubated for 2 h at room temperature in 100ul NaCl/Pi/BSA containing various concentrations of free lectins. Aliquots were then added onto A549 fixed cells in microtiter well plate. After 1 h incubation at room temperature, the plates were washed 12 times with NaCl/Pi, and the bound phages were eluted with 100 µl of 0.1 M glycine/HCl, pH 2.2. The phages were quantitated by titering of log-phase *Escherichia coli.* Isolated phage will be sequenced. To observe the binding of soluble Fabs on live cells, A549 cell immunostaining and competition assays with lectins will be performed on ice to prevent uptake of Fab fragments by the cells.

### Isolation of unique binding clones of individual phages

The obtained phages were further analyzed by competition with the specific lectin and the corresponding sugar ligand on the binding of Human A549 cancer cells that were fixed with or without specific lectins. Using this methodology we have isolated several clones with the required properties:

The following table represents the specificity binding of each phage that was isolated from the library as having binding properties as the lectin. Each phage was analyzed for its competition binding with the UEA lectin and corresponding sugar molecule (fucose). The numbers represent the binding intensity (by ELISA test) after subtracting the binding value to A549 cells. Columns 1-4 contain the same phages as column 5-8 and 9-12. Phages that compete only with the sugar moiety and not with the specific lectin are of less interest. Phages that are displaced mainly by lectins are of most interest. These binding characteristics relate to the binding affinity of the isolated phages.

We have chosen phages from Plate I, a phage at position E4 (IE4), and from plate II, phages in positions B4 (IIB4), D4 (IID4) and E4 (IIE4).

| **Plate I** | | | | | | | | | | | | |
|---|---|---|---|---|---|---|---|---|---|---|---|---|
| | **Binding and competition with UEA** | | | | **Binding with no competition** | | | | **Binding and competition with sugar** | | | |
| | **1** | **2** | **3** | **4** | **5** | **6** | **7** | **8** | **9** | **10** | **11** | **12** |
| **A** | 0 | **-0.139** | **-0.029** | **-0.266** | **-0.079** | **-0.054** | **-0.139** | **0.007** | 0.01 | 0.146 | 0.154 | **0.004** |
| **B** | **-0.064** | **-0.18** | **-0.002** | **-0.214** | **-0.237** | **-0.27** | **-0.11** | **0.001** | **0.002** | 0.535 | **0.001** | 0 |
| **C** | **0.046** | **-0.003** | **-0.206** | **-0.073** | **-0.411** | **-0.003** | **-0.01** | **0** | 0.082 | 0.49 | 0.184 | **0.009** |
| **D** | **-0.108** | **-0.085** | **-0.064** | **-0.077** | **-0.545** | **-0.186** | **-0.045** | **-0.003** | 0.173 | 0.426 | 0.305 | **-0.007** |
| **E** | **-0.01** | **-0.007** | **-0.146** | 0.119 | **-0.433** | **-0.107** | **-0.342** | -0.023 | 0.367 | **-0.004** | **-0.014** | **-0.006** |
| **F** | **0.017** | **0.001** | **0** | **-0.002** | **-0.233** | **-0.012** | **0.01** | **0** | **0.003** | **0.039** | **0.011** | **0.006** |
| **G** | **0.004** | **0.004** | **-0.007** | **-0.005** | **-0.003** | **-0.012** | **-0.009** | -0.014 | 0.008 | **-0.001** | **0** | **-0.003** |
| **H** | **0.003** | **0.001** | **-0.002** | **0.005** | 0.013 | **-0.02** | **-0.005** | **-0.009** | **0.003** | **-0.023** | **0.001** | **-0.001** |

| **Plate II** | | | | | | | | | | | | |
|---|---|---|---|---|---|---|---|---|---|---|---|---|
| | **Binding and competition with UEA** | | | | **Binding with no competition** | | | | **Binding and competition with sugar** | | | |
| | **1** | **2** | **3** | **4** | **5** | **6** | **7** | **8** | **9** | **10** | **11** | **12** |
| **A** | **-0.1** | **0.008** | **0.018** | **0.09** | **-0.219** | **0.013** | **-0.616** | **0.008** | -0.024 | 0.129 | 0.147 | **0.002** |
| **B** | **-0.148** | 0.466 | **0.039** | 0.279 | **-0.264** | 0.168 | **-0.224** | -0.014 | **-0.014** | 0.499 | **-0.004** | **-0.003** |
| **C** | **-0.109** | 0 | **0.061** | 0.171 | **-0.341** | 0 | **-0.017** | **-0.003** | -0.38 | 0.38 | 0.192 | **0.006** |
| **D** | **-0.212** | 0.535 | **0.011** | 0.121 | **-0.182** | 0.173 | **-0.023** | **-0.006** | **-0.267** | 0.277 | 0.311 | **-0.004** |
| **E** | **-0.001** | **0.012** | **0.06** | 0.273 | **-0.068** | 0.414 | **-0.144** | **-0.009** | -0.244 | **-0.001** | **-0.003** | **-0.002** |
| **F** | 0.01 | **0.007** | **0.001** | **-0.004** | **-0.047** | 0.041 | 0.012 | **0.005** | **-0.004** | 0.036 | 0.01 | **0.004** |
| **G** | 0.003 | **0.083** | **0** | **-0.003** | **-0.002** | 0.011 | -0.001 | **0.001** | **0.001** | **0.003** | **-0.002** | -0.004 |
| **H** | 0.009 | **0.009** | **0.006** | **0.004** | 0.014 | **0.002** | -0.015 | -0.013 | **-0.002** | **-0.002** | **-0.008** | -0.006 |

### Cell death validation of LICAs for NSCLC

Selected LICAs were analyzed for inducing cell death in an A549 human NSCLC model. An example of cell death induced by such isolated scFv clones is given in figure 12. Figure 12 shows growth inhibition by scFv isolated IIE4, IID4, IIB4 & IE4 selected fragments displaced by the UEA lectin. The fragments were cross-linked with secondary antibodies (anti maltose binding sequence tagged antibodies) to generate bivalent fragments.

The results demonstrated that a monovalent scFv fragment does not induce apoptosis. Upon cross-linking with secondary antibody (anti-maltose binding protein) there is a killing affect. A negative scFv fragment that is cross-linked with the secondary antibody, does not induce cell death.

From the above results, it seems that scFv fragments IIE4 and IID4 induce cell killing. We have monitored their killing affect by titrating the secondary antibody, the anti maltose binding protein (MBP) antibody (Ab), figures 13A and 13B. The results demonstrated that 1:1 ratio of secondary antibody with the scFv fragments generates a bivalent binder that induces cell apoptosis.

The LICAs will be analyzed also in the PEGylated form and in conjugate with lipid vesicles. We will also analyze the stability of the molecules in mice serum. Dose dependent experiments will be performed to determine ID₅₀, and to estimate the specificity and concentration to be used in animal studies.

### Staining of human NSCLC specimens with scFv fragment IIE4

Human NSCLC paraffin embedded tissue were stained with scFv fragment IIE4 using secondary anti maltose binding protein conjugated to Cy3, figures 14A, 14B and 14C. The results demonstrated that adenocarcinoma of the lung is positive for staining with scFv IIE4 fragment while no staining appears in neighboring normal tissue.

Figures 15A-15E, show several examples of human normal tissue array paraffin embedded slides that were stained with scFv fragment IIE4 using secondary anti maltose binding protein conjugated to Cy3, or with PNA or EUA lectins conjugated to Cy3. DAPI staining was used to identify the tissue. The results demonstrated that PNA and EUA lectins stain normal tissue in different pattern. But for the staining of gallbladder tissue, staining of normal tissues with scFv fragment IIE4 demonstrated negative staining.

The following normal human organs were stained with scFv IIE4:

| Tissue | Code | Age | Sex |
|---|---|---|---|
| **BREAST** | | | |
| breast, epithelium | BE | 46 | F |

| CARDIOVASCULAR | | | |
|---|---|---|---|
| aorta, smooth muscle | CASM | 27 | M |
| heart, myocardium | CHM | 59 | M |
| lymphatic endothelium | CLE | 27 | M |
| small muscular artery (lung) | CSMA | 56 | M |
| small vein (intestine) | CSV | 49 | M |

| ENDOCRINE | | | |
|---|---|---|---|
| adrenal gland, cortex | EAGC | unk | M |
| adrenal gland, medulla | EAGM | unk | M |
| parathyroid adenoma | EPAD | 59 | M |
| pituitary, anterior | EPA | 78 | F |
| pituitary, posterior | EPP | 78 | F |
| thyroid | ET | 48 | F |
| GASTROINTESTINAL TRACT | | | |
| esophagus, squamous mucosa | GIE | 81 | M |
| gastric mucosa, antral | GIGA | 43 | M |
| gastric mucosa, oxyntic | GIGO | 53 | F |
| small intestine, mucosa | GISI | 45 | M |
| small intestine, mucosa | GISI | 45 | F |
| colon, mucosa | GIC | 59 | F |
| anus, mucosa | GIA | 59 | M |

| GENITAL TRACT, FEMALE | | | |
|---|---|---|---|
| ectocervix | GFEC | 55 | F |
| endocervix | GFEN | 60 | F |
| endometrium, secretory | GFES | 49 | F |
| fallopian tube | GFFT | 52 | F |
| ovary, 1° oocytes | GFOO | 18 | F |
| ovary, corpus luteum | GFOCL | 49 | F |
| ovary, epithelium | GFOE | 51 | F |
| ovary, stroma | GFOS | 51 | F |

| GENITAL TRACT, MALE | | | |
|---|---|---|---|
| seminiferous tubules | GMST | 72 | M |
| epididymis | GME | 83 | M |
| seminal vessicle | GMSV | 56 | M |
| prostate | GMP | 41 | M |
| HEPATIC & PANCREATIOBILIARY | | | |
| gallbladder | HPG | 69 | M |
| liver | HPL | 58 | F |
| liver | HPL | 71 | M |
| pancreas | HPP | 43 | M |
| pancreas | HPP | 63 | M |
| pancreas | HPP | 45 | F |

| LYMPHOID | | | |
|---|---|---|---|
| lymph node | LLN | 76 | F |
| mucosa assoc. lymphoid tissue, appendix | LMALT | 17 | F |
| spleen | LS | 52 | M |
| thymus | LT | 32 | F |
| tonsil | LTL | 4 | M |
| NERVOUS SYSTEM, CENTRAL | | | |
| cerebral cortex | NCCC | 78 | F |
| cerebellar cortex, purkinje/granular layer | NCPG | 78 | F |
| choroid plexus | NCCP | 78 | F |
| ependymal cells | NCE | 78 | F |
| hippocampus | NCH | 78 | F |
| meninges | NCM | 78 | F |
| motor neurons (spinal cord) | NCMN | 78 | F |
| white matter (subcortical) | NCWM | 78 | F |
| NERVOUS SYSTEM, PERIPHERAL | | | |
| autonomic ganglia & nerves, intestinal | NPAG | 52 | M |
| peripheral nerve | NPPN | 62 | M |

| ORAL, SALIVARY & NASAL | | | |
|---|---|---|---|
| salivary gland (parotid) | OSSG | 65 | F |
| tonsil, squamous epithelium | OSTSE | 8 | F |

| PLACENTA | | | |
|---|---|---|---|
| amniotic membrane | PAM | 23 | F |
| placenta, villi | PV | 23 | F |

| RESPIRATORY TRACT | | | |
|---|---|---|---|
| alveoli | RA | 73 | M |
| bronchus, epithelium | RBE | 73 | M |
| bronchus, epithelium | RBE | 16 | M |

| SKIN | | | |
|---|---|---|---|
| skin, squamous epithelium | SSE | 60 | F |
| subepidermal tissue | SST | 60 | F |
| subepidermal tissue | SST | 62 | M |

| SOFT TISSUE | | | |
|---|---|---|---|
| adipose tissue, breast | STAB | 39 | F |
| cartilage, articular | STCA | 62 | M |
| cartilage, bronchial | STCB | 79 | M |
| skeletal muscle | STSKM | 49 | M |
| smooth muscle, intestine | STSMI | 52 | M |
| smooth muscle, uterus | STSMU | 55 | F |
| synovium | STS | 62 | M |

| UROLOGICAL TRACT | | | |
|---|---|---|---|
| kidney, cortex | UKC | 62 | M |
| kidney, medulla | UKM | 91 | M |
| bladder, transitional epithelium | UBTE | 41 | M |

### Efficacy of LICAs in animal model

In preliminary experiments, we had determined the growth rate of tumors followed injection of human NSCLC H1299 and A549 cells in CD1nude mice. Cells will be injected into the flanks of CD 1 nude mice, and when the tumor reaches a size of 20-30 mm³ (approximately 5 days) the antibodies will be injected into the tumor. Good results are at least 25% reduction in tumor size with this approach (at a SD of 10%). Assuming a significance of 5% and a study power of 90%. In order to obtain a wide safety margin (animal death etc.) 10 mice per treatment group will be used. The following table, based on preliminary data, describes the animal groups, the cells to be injected, for one LICA to be used. LICAs (approximately 50µg/100µl PBS/injection, dose A - 2mg/Kg, and 250µg/100µl PBS/injection, dose B - 10mg/Kg) will be injected directly into the tumor or intravenously. The selected LICA will be injected every three days up to 5 times. Table 8 shows the arms of an exemplary experiment.

**Table 8: Mice per group for dose A and B**

| Cells | H1299 | A549 | non |
|---|---|---|---|
| Injection (IV or IT) | | | |
| PBS - no LICA | 4 | 4 | 4 |
| LICA-1 | 10 | 10 | 4 |
| LICA-2 | 10 | 10 | 4 |
| LICA-3 | 10 | 10 | 4 |

Tumor parameters to be scored following therapy include:
Size of tumors over time in the different experimental groups; existence of metastases;
level of cell death or apoptosis by Annexin V staining and TUNEL assays in the remaining tumors; level of proliferation by scoring for mitotic indices, Ki-67 and cyclin D1 protein levels in the remaining tumors.

Tables 9 and 10 hereinbelow provide non-limiting example of non-mammalian lectins that may be utilized in the methods of the present invention.

**Table 9**

| **Lectin Abbrev.** | **Organism** | **Glycan Affinity** |
|---|---|---|
| ABA | *Agaricus bisporus* | Fetuin; Galβ1-3GalNAc |
| ACL | *Amaranthus caudatus* | Galβ 1-3GalNAc, Neu5Acα2-3Galβ 1-3GalNAc; T-Antigen |
| GSLI | *Griffonia simplicifolia lectin I* | α-N-acetylgalactosamine, α-galactose |
| GSL II | *Griffonia simplicifolia* | terminal-α,β-GlcNAc; glycogen |
| GSL I B4 | *Griffonia simplicifolia* | α-D-galactosyl residues |
| BPL | *Bauhinia purpurea alba* | Galβ1-3GalNAc |
| CFL | *Codium fragile* | GalNAc |
| DSL | *Datura stramonium* | (GlcNAcβ1-4)₃GlcNAc = (Glcβ1-4)₂GlcNAc > Glcβ1-4GlcNAc >> GlcNAc |
| DBA | *Dolichos biflorus* | terminal FP > GalNAcα1-3GalNAc > GalNAcα1-3Gal; blood group A_{I} (Forssman pentasaccharide: GalNAcα1-3GalNAcα1-3Galβ1-4Galβ1-4GlcNAc) |
| ECor A | *Erythrina coralldendron* | GalNAc/N-acetyllactosamin/Lactose/D-Gal |
| EEA | *Euonymos europaeus* | Galα1-3(L-Fucα1-2)Galβ1,3/4-β -GlcNAc; Galal-3Gal; blood group H structures |
| HAA | *Hel ix aspersa* | terminal αGalNAc residues |
| HPA | *Helix pomatia* | GalNAcα1-3GalNAc > α-GalNAc > α-GlcNAc » α-Gal |
| HHL | *Hippeastrum hybrid* | (α1,3)/(α1,6) mannose; polymannose structures; yeast galactomannans |
| LTL | *Lotus tetragonolobus* | α-L-fucose |
| LEL | *Lycopersicon escul entum* | (GlcNAcβ 1-4)₃ GlcNAc > (GlcNAcβ1-4)₂ GlcNAc > GlcNAcβ1-4GlcNAc |
| MPA | *Maclura pomifera* | terminal Galβ1-3GalNAc > GalNAcα 1-6Gal |
| NPA | *Narcissus pseudonarcissus* | terminal and internal α-D-mannosylresidues on glycoconjugates, preferably oligomannoses containing α1-6 linkages |
| PCA | *Phaseolus coccineus* | agglutination is not inhibited by monosaccharides but is inhibited by fetuin |
| PHA-L | *Phaseolus vulgaris L* | GlcNAcβ1,2Man, triantennary complex oligosaccharides |
| PHA-E | *Phaseolus vulgaris E* | Galβ1,4GlcNAcβ1,2Manα1,6 |
| PWM | *Phyto*/*acca americana* | N-acetyl-β-D-glucosamine oligomers |
| PSA, PEA | *Pisum sativum* | branched α-man, complex type with N-acetylchitobiose-linked core α-fuc |
| PTL, WBA | *Psophocarpus tetragonolobus I* | α-galactosamine |
| SBA | *Glycine max* | terminal α,βGalNAc > α,βGal |
| STA | *Solanum tuberosum* | N-acetyl-β-D-glucosamine oligomers |
| SJA | *Sophora japonica terminal* | Galβ1,3GalNAc>Galβ 1,3GlcNAc>αβ,GalNAc>αβ,Gal |
| WFA,WFL | *Wisteria floribunda* | terminal N-acetylgalactosamine-α- or β-3 or 6-galactose |

**Table 10**

| Lectin Abbrev. | Lectin Full Name | Organism | Glycan Affinity |
|---|---|---|---|
| Mannose binding lectins | | | |
| Con A | Concanavalin A | Canavalia ensiformis | branched α -mannosidic structures; |
| | | | high-mannose type, hybrid type and biantennary complex type N-Glycans |
| LCH | Lentil lectin | Lens culinaris | Fucosylated core region of bi- and triantennary complex type N-Glycans |

| GNA | Snowdrop lectin | Galanthus nivalis | α 1-3 and α 1-6 linked high mannose structures |
|---|---|---|---|
| Galactose / N-acetylgalactosamine binding lectins | | | |
| RCA | Ricinus communis Agglutinin, RCA₁₂₀ | Ricinus communis | Galβ1-4GlcNAcβ1-R |
| ECL | Coral Tree | Erythrina cristagalli | Galβ1-4GlcNAcβ1-R |
| PNA | Peanut Agglutinin | Arachis hypogaea | Galβ1-3GalNAcα1 -Ser/Thr (T-Antigen) |
| AIL | Jacalin | Artocarpus integrifolia | (Sia)Galβ1-3GalNAcα1-Ser/Thr (T-Antigen) |
| VVL or VVA | Hairy vetch lectin | Vicia villosa | GalNAcα-Ser/Thr (Tn-Antigen) |

| Sialic acid / N-acetylglucosamine binding lectins | | | |
|---|---|---|---|
| WGA | Wheat Germ agglutinin | Triticum vulgaris | GlcNAcβ1-4GlcNAcβ1-4GlcNAc, Neu5Ac (sialic acid) |
| SNA | Elderberry lectin | Sambucus nigra | Neu5Acα2-6Gal(NAc)-R |
| MAL II or MAA | Maackia amurensis lectin | Maackia amurensis | Neu5Ac/Gcα2-3Galβ1-4GlcNAcβ1-R |

| Fucose binding lectins | | | |
|---|---|---|---|
| UEA | Ulex europaeus agglutinin | Ulex europaeus | Fucα1-2Gal-R |
| AAL | Aleuria aurantia lectin | Aleuria aurantia | Fucα1-2Galβ1-4(Fucα1-3/4)Galβ1-4GlcNAc; R₂-GlcNAcβ1-4(Fucα 1-6)GlcNAc-R₁ |

### References

Azriel-Rosenfeld R, Valensi M, Benhar I (2004) A Human synthetic combinatorial library of arrayable single-chain antibodies based on shuffling in vivo formed CDRs into general framework regions. J Mol. Biol. 335: 177-92.
Benhar I, (2001) Biotechnological applications of phage and cell display. Biotech. Advances 19: 1-33.
Blixt O, Head S, Mondala T, Scanlan C, Huflejt ME, Alvarez R, Bryan MC, Fazio F, Calarese D, Stevens J, Razi N, Stevens DJ, Skehel JJ, van Die I, Burton DR, Wilson IA, Cummings R, Bovin N, Wong CH, Paulson JC (2004) Printed covalent glycan array for ligand profiling of diverse glycan binding proteins. PNAS USA. 101(49):17033-8.
Cai, X and Garen A. (1995) Anti-melanoma antibodies from melanoma patients immunized with genetically modified autologous tumor cells: selection of specific antibodies from single-chain Fv fusion phage libraries. PNAS USA 3;92(14):6537-41.
Carlow DA, Williams MJ, Ziltener HJ. (2003) Modulation of O-Glycans and N-Glycans on Murine CD8 T Cells Fails to Alter Annexin V Ligand Induction by Galectin. J. Immunology 171(10):5100-6.
Fukukawa T, Matsuzaki H, Haruta Y, Hara H, Seon BK. (1986) New monoclonal antibodies SN3, SN3a and SN3b directed to sialic acid of glycoprotein on human non-T leukaemia cells. Exp. Hematol. 14: 850-855.
Fuster, MM and Esko JD. (2005). The Sweet and Sour of Cancer: Glycans as Novel Therapeutic Targets. Nat Rev Cancer. 5(7):526-542.
Gastman B, Wang K, Han J, Zhu ZY, Huang X, Wang GQ, Rabinowich H, Gorelik E. (2004). A novel apoptotic pathway as defined by lectin cellular initiation, BBRC 316(1):263-71.
Gorelik E., Galili U, Raz A (2001) On the role of cell surface carbohydrates and their binding proteins (lectins) in tumor metastasis. Cancer Metastasis Rev. 20:245-277.
Griffiths AD, Malmqvist M, Marks JD, Bye JM, Embleton MJ, McCafferty J, Baier M, Holliger KP, Gorick BD, Hughes-Jones NC, et al. (1993) Human anti-self antibodies with high specificity from phage display libraries. EMBO J. 12(2): 725-734.
Higuchi Mitsunori (2003) Identification of decay-accelerating factor (DAF) as a peanut aggutinin (PNA) lectin-binding molecule in human lung and its down-regulation in non-small cell lung cancers (NSCLC). Proceedings of the American association for cancer research annual meeting. Vol. 44, July 2003, page 999, XP008075831.
Houghton AN, Mintzer D, Cordon-Cardo C, Welt S, Fliegel B, Vadhan S, Carswell E, Melamed MR, Oettgen HF, Old LJ. (1985) Mouse monoclonal IgG3 antibody detecting GD3 ganglioside: a phase I trial in patients with malignant melanoma. PNAS U S A. 82(4):1242-6.
Hu R, Zhai Q, Liu W, Liu X. (2001) An insight into the mechanism of cytotoxicity of ricin to hepatoma cell: roles of Bcl-2 family proteins, caspases, Ca(2+)-dependent proteases and protein kinase C. J Cell Biochem. 81(4):583-93.
Kim M, Rao MV, Tweardy DJ, Prakash M, Galili U, Gorelik E. (1993) Lectin-induced apoptosis of tumour cells. Glycobiology. 3(5):447-53.
Kornfeld KS (1980) In: Lennarz W (ed.) The Biochemistry of Glycoproteins and Proteoglycans, Plenum Press, New York).
Kramer K, Gerald WL, Kushner BH, Larson SM, Hameed M, Cheung NK. (2001) Disaloganglioside GD2 loss following monoclonal antibody therapy is rare in neuroblastoma. Med Pediatr Oncol. Jan;36(1):194-6.
Kuramoto T, Uzuyama H, Hatakeyama T, Tamura T, Nakashima T, Yamaguchi K, Oda T. (2005) Cytotoxicity of a GalNAc-specific C-type lectin CEL-I toward various cell lines. J Biochem. 137(1):41-50.
Lyu SY, Choi SH, Park WB. (2002). Korean mistletoe lectin-induced apoptosis in hepatocarcinoma cells is associated with inhibition of telomerase via mitochondrial controlled pathway independent of p53. Arch Pharm Res. 25(1):93-101
Mintz PJ, Kim J, Do KA, Wang X, Zinner RG, Cristofanilli M, Arap MA, Hong WK, Troncoso P, Logothetis CJ, Pasqualini R, Arap W. (2003) Fingerprinting the circulating repertoire of antibodies from cancer patients. Nat Biotech. 21(1):57-63.
Monzavi-Karbassi B, Artaud C, Jousheghany F, Hennings L, Carcel-Trullols J., Shaaf S, Korourian S and Kieber-Emmons, T (2005). Reduction of spontaneous metastases through induction of carbohydrate cross-reactive apoptotic antibodies. J Immunol. 174(11):7057-65.
Miyoshi N, Koyama Y, Katsuno Y, Hayakawa S, Mita T, Ohta T, Kaji K, Isemura M. (2001) Apoptosis Induction Associated with Cell Cycle Dysregulation by Rice Bran Agglutinin. J. Biochem.130:799-805.
Nissim A, Hoogenboom HR, Tomlinson IM, Flynn G, Midgley C, Lane D, Winter G. (1994) Antibody fragments from a 'single pot' phage display library as immunochemical reagents. EMBO J. 13(3):692-8.
Opdenakker G, Rudd PM, Ponting C P, Dwek R A (1993) Concepts and principles of glycobiology. FASEB J 7: 1330-1337.
Portolano S, McLachlan SM, Rapoport B. (1993) High affinity, thyroid-specific human autoantibodies displayed on the surface of filamentous phage use V genes similar to other autoantibodies. J Immunol. 1;151(5):2839-51.
Schwarz R, Wojciechowicz DC, Picon AI, Schwarz MA, Paty PB (1999) Wheatgerm agglutinin-mediated toxicity in pancreatic cancer cell. British J. Cancer 80:1754-62.
Sharon N, Lis H (1989) Lectins as cell recognition molecules. Science 246: 227-234.
Sharon, N. and Lis, H. (2004) History of lectins: from hemagglutinins to biological recognition molecules. Glycobiology 14(11):53R-62R.
Somers VA, Brandwijk RJ, Joosten B, Moerkerk PT, Arends JW, Menheere P, Pieterse WO, Claessen A, Scheper RJ, Hoogenboom HR, Hufton SE. (2002). A Panel of Candidate Tumor Antigens in Colorectal Cancer Revealed by the Serological Selection of a Phage Displayed cDNA Expression Library. J Immunology 169: 2772-2780
Zhao, C, Sun H, Tong X and Qi Y. (2003). An antitumor lectin from the edible mushroom Agrocybe aegerita. Biochem J. 374:321-327.

## Claims

1. A method for identifying a therapeutic molecule for the diagnosis or treatment of cancer, the method comprising the steps of:
a. providing a lectin which selectively binds to a tumor-associated carbohydrate antigen on a tumor cell and induces death of the tumor cell;
b. providing a library of candidate molecules;
c. contacting the tumor cell with the lectin under conditions that allow binding between the lectin and said tumor-associated carbohydrate antigen;
d. contacting the tumor cell of (c) with the library of candidate molecules under conditions suitable to displace the lectin; and
e. identifying at least one candidate molecule, which is capable of displacing the lectin.

2. A method for identifying a therapeutic molecule for the diagnosis or treatment of cancer, the method comprising the steps of:
a. providing a lectin which selectively binds to a tumor associated carbohydrate antigen on a tumor cell and induces death of the cell;
b. providing a library of candidate molecules;
c. contacting the tumor cell with the library of candidate molecules under conditions that allow binding between a candidate molecule and said tumor associated carbohydrate antigen;
d. contacting the tumor cell of (c) with a lectin under conditions to displace the candidate molecule;
e. identifying the candidate molecule in the displaced fraction of (d); and optionally
f. isolating the candidate molecule.

3. The method according to claim 1 or 2, wherein the library of candidate molecules is selected from the group consisting of a phage display library, a peptide library and a small compound library.

4. The method according to claim 3,
wherein the library of candidate molecules is selected from the group consisting of an antibody phage display library and a peptide phage display library, preferably wherein the phage of the antibody phage display library displays a molecule comprising at least the antigen binding portion of an antibody, more preferably wherein the antibody fragment is selected from a scFv and a Fab fragment; or
wherein the library is a peptide library, preferably wherein the peptide library is a peptide analog library.

5. The method according to claim 1 or 2,
wherein the tumor associated carbohydrate antigen is selected from the group consisting of a glycosaminoglycan, a glycoprotein and a glycolipid, preferably wherein the tumor associated carbohydrate antigen comprises a carbohydrate moiety selected from the group consisting of sialic acid, an N-linked carbohydrate, and O-linked carbohydrate and combinations thereof; or
wherein the lectin is a non-mammalian lectin; or
wherein the therapeutic molecule further comprises an imaging agent.

## Patentansprüche

1. Verfahren zur Identifizierung eines therapeutischen Moleküls zur Diagnose oder Behandlung von Krebs, welches die Schritte umfasst:
a. Bereitstellen eines Lectins, das selektiv an ein Tumor-assoziiertes Kohlenhydrat-Antigen auf einer Tumorzelle bindet und den Tod der Tumorzelle induziert,
b. Bereitstellen einer Bank mit Kandidaten-Molekülen,
c. Inkontaktbringen der Tumorzelle mit dem Lectin unter Bedingungen, die eine Bindung zwischen dem Lectin und dem Tumor-assoziierten Kohlenhydrat-Antigen ermöglicht,
d. Inkontaktbringen der Tumorzelle von (c) mit der Kandidaten-Molekül-Bank unter Bedingungen, die geeignet sind, das Lectin zu ersetzen, und
e. Identifizieren von mindestens einem Kanditaten-Molekül, das in der Lage ist, das Lectin zu ersetzen.

2. Verfahren zur Identifizierung eines therapeutischen Moleküls zur Diagnose oder Behandlung von Krebs, welches die Schritte umfasst:
a. Bereitstellen eines Lectins, das selektiv an ein Tumor-assoziiertes Kohlenhydrat-Antigen auf einer Tumorzelle bindet und den Tod der Tumorzelle induziert,
b. Bereitstellen einer Bank mit Kandidaten-Molekülen,
c. Inkontaktbringen der Tumorzelle mit der Kandidaten-Molekül-Bank unter Bedingungen, die eine Bindung zwischen einem Kandidaten-Molekül und dem Tumor-assoziierten Kohlenhydrat-Antigen ermöglicht,
d. Inkontaktbringen der Tumorzelle von (c) mit einem Lectin unter Bedingungen, die geeignet sind, das Kandidaten-Molekül zu ersetzen,
e. Identifizieren des Kanditaten-Moleküls in der ersetzten Fraktion von (d), und wahlweise,
f. Isolieren des Kandidaten-Moleküls.

3. Verfahren nach Anspruch 1 oder 2, wobei die Kandidaten-Molekül-Bank ausgewählt ist der Gruppe bestehend aus einer Phagen-Darbietungs-Bank, einer Peptidbank und einer Bank kleiner Verbindungen.

4. Verfahren nach Anspruch 3,
wobei die Kandidaten-Molekül-Bank ausgewählt ist aus der Gruppe bestehend aus einer Antikörper-Phagen-Darbietungs-Bank und einer Peptid-Phagen-Darbietungs-Bank, vorzugsweise wobei der Phage der Antikörper-Phagen-Darbietungsbank ein Molekül zeigt, das mindestens den Antigen-bindenden Bereich eines Antikörpers umfasst, mehr bevorzugt wobei das Antikörper-Fragment ausgewählt ist unter einem scFv und einem Fab-Fragment, oder
wobei die Bank eine Peptidbank ist, vorzugsweise wobei die Peptidbank eine PeptidAnalogbank ist.

5. Verfahren nach Anspruch 1 oder 2,
wobei das Tumor-assoziierte Kohlenhydrat-Antigen ausgewählt ist aus der Gruppe bestehend aus einem Glycosaminglycan, einem Glycoprotein und einem Glycolipid, vorzugsweise wobei das Tumor-assoziierte Kohlenhydrat-Antigen eine Kohlenhydratgruppe umfasst, ausgewählt aus der Gruppe bestehend aus einer Sialinsäure, einem N-verknüpften Kohlenhydrat, und einem O-vernüpften Kohlenhydrat und Kombinationen davon, oder
wobei das Lectin ein nicht-Säuger-Lectin ist, oder
wobei das therapeutische Molekül weiter ein Bild-gebendes Mittel umfasst.

## Revendications

1. Procédé d'identification d'une molécule thérapeutique pour le diagnostic ou le traitement d'un cancer, le procédé comprenant les étapes consistant à :
a. fournir une lectine qui se lie de façon sélective à un antigène de glucide associé à une tumeur sur une cellule tumorale et induit la mort de la cellule tumorale ;
b. fournir une banque de molécules candidates ;
c. mettre la cellule tumorale en contact avec la lectine dans des conditions qui permettent une liaison entre la lectine et ledit antigène de glucide associé à une tumeur ;
d. mettre la cellule tumorale de (c) en contact avec la banque de molécules candidates dans des conditions appropriées pour déplacer la lectine ; et
e. identifier au moins une molécule candidate, qui est capable de déplacer la lectine.

2. Procédé d'identification d'une molécule thérapeutique pour le diagnostic ou le traitement d'un cancer, le procédé comprenant les étapes consistant à :
a. fournir une lectine qui se lie de façon sélective à un antigène de glucide associé à une tumeur sur une cellule tumorale et induit la mort de la cellule ;
b. fournir une banque de molécules candidates ;
c. mettre la cellule tumorale en contact avec la banque de molécules candidates dans des conditions qui permettent une liaison entre une molécule candidate et ledit antigène de glucide associé à une tumeur ;
d. mettre la cellule tumorale de (c) en contact avec la lectine dans des conditions pour déplacer la molécule candidate ; et
e. identifier la molécule candidate dans la fraction déplacée de (d) ; et éventuellement
f. isoler la molécule candidate.

3. Procédé selon la revendication 1 ou la revendication 2, dans lequel la banque de molécules candidates est sélectionnée parmi le groupe constitué d'une banque d'affichage de phages, d'une banque de peptides et d'une banque de petits composés.

4. Procédé selon la revendication 3,
dans lequel la banque de molécules candidates est sélectionnée parmi le groupe constitué d'une banque d'affichage de phages d'anticorps et d'une banque d'affichage de phage de peptides, de préférence dans lequel le phage de la banque d'affichage de phages d'anticorps affiche une molécule comprenant au moins la portion de liaison à l'antigène d'un anticorps, plus préférablement dans lequel le fragment d'anticorps est sélectionné parmi un fragment scFv et Fab ; ou
dans lequel la banque est une banque de peptides, de préférence dans lequel la banque de peptides est une banque de peptides analogues.

5. Procédé selon la revendication 1 ou la revendication 2,
dans lequel l'antigène de glucide associé à une tumeur est sélectionné parmi le groupe constitué d'un glycosaminoglycan, d'une glycoprotéine et d'un glycolipide, de préférence dans lequel l'antigène de glucide associé à une tumeur comprend une fraction glucidique sélectionnée parmi le groupe constitué d'acide sialique, d'un glucide lié par N, et d'un glucide lié par O et des combinaisons de ceux-ci ; ou
dans lequel la lectine est une lectine non de mammifère ; ou
dans lequel la molécule thérapeutique comprend en outre un agent d'imagerie.
